(19) Europäisches Patentamt / European Patent Office / Office européen des brevets

(11) **EP 4 105 336 B1**

(12) # EUROPEAN PATENT SPECIFICATION

(45) Date of publication and mention of the grant of the patent:
**29.07.2026 Bulletin 2026/31**

(21) Application number: **21753771.1**

(22) Date of filing: **15.02.2021**

(51) International Patent Classification (IPC):
***C12Q 1/66*** *(2006.01)* ***C12Q 1/00*** *(2006.01)*
*G01N 21/76* *(2006.01)*

(52) Cooperative Patent Classification (CPC):
**C12Q 1/66; C12Q 1/008; C12Y 113/12007;**
G01N 21/763

(86) International application number:
**PCT/JP2021/005403**

(87) International publication number:
**WO 2021/162123 (19.08.2021 Gazette 2021/33)**

(54) **LIQUID COMPOSITION FOR MEASURING ATP, AND AMP AND/OR ADP IN A SAMPLE**

FLÜSSIGE ZUSAMMENSETZUNG ZUR MESSUNG VON ATP SOWIE AMP UND/ODER ADP IN EINER PROBE

COMPOSITION LIQUIDE POUR MESURER L'ATP ET L'AMP ET/OU L'ADP DANS UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2020 JP 2020023442**

(43) Date of publication of application:
**21.12.2022 Bulletin 2022/51**

(73) Proprietor: **Kikkoman Corporation**
**Noda-shi, Chiba 278-8601 (JP)**

(72) Inventors:
- **SUZUKI Shigeya**
  **Noda-shi, Chiba 278-8601 (JP)**
- **ICHIYANAGI Yuko**
  **Noda-shi, Chiba 278-8601 (JP)**

(74) Representative: **J A Kemp LLP**
**80 Turnmill Street**
**London EC1M 5QU (GB)**

(56) References cited:

**WO-A1-01/81618** **WO-A1-2017/057672**
**WO-A1-99/11816** **JP-A- 2001 204 496**
**US-A- 5 891 659** **US-A1- 2020 024 638**

- **SAKAKIBARA T. ET AL.: "Enumeration of bacterial cell numbers by amplified firefly bioluminescence without cultivation", ANAL. BIOCHEM., vol. 312, no. 1, 2003, pages 48 - 56, XP055411343**
- **BAKKE M. ET AL.: "Development of a novel hygiene monitoring system based on the detection of total adenylate (ATP+ADP+AMP)", J. FOOD PROT., vol. 81, no. 5, 3 April 2018 (2018-04-03), pages 729 - 737, XP093136435**
- **BROVKO L.Y. ET AL.: "Bioluminescent assay of bacterial intracellular AMP, ADP, and ATP with the use of a coimmobilized three-enzyme reagent (adenylate kinase, pyruvate kinase, and firefly luciferase)", ANAL. BIOCHEM., vol. 220, no. 2, August 1994 (1994-08-01), pages 410 - 414, XP024763212**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 105 336 B1

## Description

### Technical Field

In one embodiment, the present invention relates to the use of a liquid composition for measuring ATP and AMP and/or ADP in a sample after storage of the liquid composition, and a method for measuring ATP and AMP and/or ADP in a sample, comprising using the liquid composition.

### Background Art

Adenosine triphosphate (hereinafter, referred to as ATP) is a nucleotide found in various organisms and is utilized by cells as a substrate that stores or releases energy. Since ATP is included in biogenic substances, a kit and a method for measuring cleanliness of a bio-related sample and a bio-related instrument and a kit for measuring contamination of a cooking-related instrument, through the use of ATP measurement and the like have been reported (Patent Literatures 1 and 2).

Known representative methods for measuring ATP include a method comprising causing a reaction of ATP and the substrate luciferin in the presence of luciferase and measuring luminescence (Non Patent Literature 1). This reaction is catalyzed by luciferase and occurs in the presence of a divalent metal ion as follows.

Luciferin + ATP + $O_2 \rightarrow$ oxyluciferin + adenosine monophosphate (AMP) + pyrophosphoric acid (PPi) + $CO_2$ + light

ATP can be relatively readily dephosphorylated into ADP, and ADP can also be dephosphorylated into AMP in some cases. Thus, the measurement, not of ATP alone, but rather of ATP and ADP or ATP, ADP and AMP allows stable measurement of ATP (or degradation products thereof) contained in a biogenic substance and more accurate determination of cleanliness. For example, Patent Literature 1 states that the accurate detection of blood remaining in or attached to blood-related instruments or the like is enabled not by the measurement of ATP alone but rather by the measurement of ATP and ADP or ATP, ADP and AMP.

### Citation List

#### Patent Literature

Patent Literature 1: International Publication No. WO 2018/147442
Patent Literature 2: International Publication No. WO 2018/147443

#### Non Patent Literature

Non Patent Literature 1: Marlene DeLuca, William D. McElroy, Biochemistry, 1974, 13 (5), pp 921-925

### Summary of Invention

#### Technical Problem

Luminescence reagents for use in reaction systems that measure ATP as well as ADP and/or AMP by directly or indirectly converting AMP to ATP (cycling reaction) are referred to as "cycling luminescence reagents". Both of the existing cycling luminescence reagents LuciPac Pen (ATP + AMP measurement) (manufactured by Kikkoman Biochemifa Company) and LuciPac A3 (ATP + ADP + AMP measurement) (manufactured by Kikkoman Biochemifa Company) are designed and produced in a powder state, and liquid cycling luminescence reagents do not exist. However, since the production of powder reagents requires steps such as drying and pulverization and since rapid and accurate filling is difficult, such a production method is complicated and costly. Furthermore, powders also have the disadvantage that measures against moisture absorption, etc. are necessary.

Accordingly, the present inventors have newly attempted to design a liquid cycling luminescence reagent. Further, the present inventors have newly found that liquid cycling luminescence reagents are not always sufficiently stable.

In one embodiment, an object of the present invention is to provide a liquid cycling luminescence reagent

excellent in stability. In one embodiment, an object of the present invention is to provide a method for measuring ATP and AMP and/or ADP in a sample using the cycling luminescence reagent

Solution to Problem

The present inventors have found that, surprisingly, in cycling luminescent reaction using luciferin-luciferase reaction, the stability of a liquid cycling luminescence reagent is improved by reducing the relative luminescence level of the liquid cycling reagent during storage.

The present invention encompasses the following embodiments.

[1] Use of a liquid composition for measuring ATP and AMP and/or ADP in a sample after storage of the liquid composition, wherein the liquid composition has been stored one day or longer, and wherein

(i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
(ii) at least one or more of the following conditions are satisfied:

the concentration of luciferin in the liquid composition is 0.4 mM or lower;
the concentration of luciferase in the liquid composition based on the Bradford method is 0.3 mg/mL or lower;
the concentration of the enzyme that catalyzes a reaction that produces ATP from AMP in the liquid composition is 1 U/mL or lower;
the concentration of the substrate of the enzyme that catalyzes a reaction that produces ATP from AMP in the liquid composition is 0.1 mM or lower; and
the concentration of the cofactor in the liquid composition is 6 mM or lower.

[2] The use according to [1], wherein the liquid composition further comprises at least one component selected from an enzyme that catalyzes a reaction that produces ATP from ADP, a substrate of the enzyme that catalyzes a reaction that produces ATP from ADP, an enzyme that catalyzes a reaction that produces AMP from ADP, and a substrate of the enzyme that catalyzes a reaction that produces AMP from ADP, or at least one of these components is added to the liquid composition before or during measurement.
[3] Use of a liquid composition for measuring ATP and AMP and/or ADP in a sample after storage of the liquid composition, wherein the liquid composition has been stored one day or longer, and wherein

(i) the liquid composition comprises luciferase, luciferin, an enzyme that catalyzes a reaction that produces ADP from AMP, a substrate of the enzyme that catalyzes a reaction that produces ADP from AMP, an enzyme that catalyzes a reaction that produces ATP from ADP, a substrate of the enzyme that catalyzes a reaction that produces ATP from ADP, and a cofactor, or when at least one of these components is not contained in the liquid composition, the component that is not contained in the liquid composition is added thereto before or during measurement, and
(ii) at least one or more of the following conditions are satisfied:

the concentration of the enzyme that catalyzes a reaction that produces ADP from AMP in the liquid composition is 450 U/mL or lower;
the concentration of the substrate of the enzyme that catalyzes a reaction that produces ADP from AMP in the liquid composition is 0.1 mM or lower;
the concentration of the enzyme that catalyzes a reaction that produces ATP from ADP in the liquid composition is 20 U/mL or lower;
the concentration of the substrate of the enzyme that catalyzes a reaction that produces ATP from ADP is 1.2 mM or lower; and
the concentration of the cofactor in the liquid composition is 6 mM or lower.

[4]The use according to [1] to [3], wherein the storage period is 30 days or longer.
[5] The use according to any of [1] to [4] , wherein the liquid composition does not contain at least one component selected from luciferase, luciferin, an enzyme that catalyzes a reaction that produces ATP from AMP, a substrate of the enzyme that catalyzes a reaction that produces ATP from AMP, an enzyme that catalyzes a reaction that produces ADP from AMP, a substrate of the enzyme that catalyzes a reaction that produces ADP from AMP, an enzyme that

catalyzes a reaction that produces ATP from ADP, a substrate of the enzyme that catalyzes a reaction that produces ATP from ADP, and a cofactor, and the component that is not contained in the liquid composition is added thereto before or during measurement.

[6] The use according to any of [1] to [5], wherein the concentration of luciferin in the liquid composition is 0.4 mM or lower, and/or the concentration of luciferase based on the Bradford method therein is 0.3 mg/mL or lower.

[7] The use according to any of [1] to [6], wherein the concentration of luciferin in the liquid composition is 0.1 mM or lower.

[8] The use according to any of [1] to [7], wherein the concentration of luciferase in the liquid composition based on the Bradford method is 0.1 mg/mL or lower.

[9] A method for measuring ATP and AMP and/or ADP in a sample, comprising bringing a liquid composition into contact with a sample and measuring the level of luminescence, wherein the liquid composition is for measuring ATP and AMP and/or ADP in a sample after storage of the liquid composition, wherein the liquid composition has been stored 1 day or longer, and wherein

(i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
(ii) at least one or more of the following conditions are satisfied:

the concentration of luciferin in the liquid composition is 0.4 mM or lower;
the concentration of luciferase in the liquid composition based on the Bradford method is 0.3 mg/mL or lower;
the concentration of the enzyme that catalyzes a reaction that produces ATP from AMP in the liquid composition is 1 U/mL or lower;
the concentration of the substrate of the enzyme that catalyzes a reaction that produces ATP from AMP in the liquid composition is 0.1 mM or lower; and
the concentration of the cofactor in the liquid composition is 6 mM or lower.

[10] The method according to [9], wherein an ATP standard solution is not used.

Advantageous Effects of Invention

In one embodiment, the present invention involves the use of a liquid cycling luminescence reagent excellent in stability.

Brief Description of Drawings

[Figure 1] Figure 1 illustrates the change of luminescence level over time of a non-cycling luminescence reagent.
[Figure 2] Figure 2 illustrates the stability of a cycling luminescence reagent containing each concentration of luciferin.
[Figure 3] Figure 3 illustrates the level of luminescence after 9 hours of the cycling luminescence reagent containing each concentration of luciferin in Figure 2.
[Figure 4] Figure 4 illustrates the stability of a cycling luminescence reagent containing or not containing luciferin.
[Figure 5] Figure 5 illustrates the stability of a non-cycling or cycling luminescence reagent. The results indicate both the case of not containing luciferin and the case of containing luciferin.
[Figure 6] Figure 6 illustrates the stability of a cycling luminescence reagent containing each concentration of luciferase.
[Figure 7] Figure 7 illustrates the level of luminescence after 9 hours of the cycling luminescence reagent containing each concentration of luciferase.
[Figure 8] Figure 8 illustrates the ATP concentration dependency of the decrease in luminescence level of a cycling luminescence reagent.
[Figure 9] Figure 9 illustrates the stability (change of luminescence level over time) of a luminescence reagent containing each concentration of luciferin when 0.01 mL of $1 \times 10^{-5}$ M ATP is added to 0.35 mL of the luminescence reagent.
[Figure 10] Figure 10 illustrates the level of luminescence, after 1 hour, of the luminescence reagent containing each concentration of luciferin when 0.01 mL of $1 \times 10^{-5}$ M ATP is added to 0.35 mL of the luminescence reagent.
[Figure 11] Figure 11 illustrates the stability (change of luminescence level over time) of a luminescence reagent

containing each concentration of luciferin when 0.01 mL of 1 × $10^{-6}$ M ATP is added to 0.35 mL of the luminescence reagent.

[Figure 12] Figure 12 illustrates the level of luminescence, after 1 hour, of the luminescence reagent containing each concentration of luciferin when 0.01 mL of 1 × $10^{-6}$ M ATP is added to 0.35 mL of the luminescence reagent.

[Figure 13] Figure 13 illustrates the stability (change of luminescence level over time) of a luminescence reagent containing each concentration of luciferin when 0.01 mL of 1 × $10^{-7}$ M ATP is added to 0.35 mL of the luminescence reagent.

[Figure 14] Figure 14 illustrates the level of luminescence, after 1 hour, of the luminescence reagent containing each concentration of luciferin when 0.01 mL of 1 × $10^{-7}$ M ATP is added to 0.35 mL of the luminescence reagent.

[Figure 15] Figure 15 illustrates the stability (change of luminescence level over time) of a luminescence reagent containing each concentration of luciferase when 0.01 mL of 1 × $10^{-5}$ M ATP is added to 0.35 mL of the luminescence reagent.

[Figure 16] Figure 16 illustrates the level of luminescence, after 1 hour, of the luminescence reagent containing each concentration of luciferase when 0.01 mL of 1 × $10^{-5}$ M ATP is added to 0.35 mL of the luminescence reagent.

[Figure 17] Figure 17 illustrates the stability (change of luminescence level over time) of a luminescence reagent containing each concentration of luciferase when 0.01 mL of 1 × $10^{-6}$ M ATP is added to 0.35 mL of the luminescence reagent.

[Figure 18] Figure 18 illustrates the level of luminescence, after 1 hour, of the luminescence reagent containing each concentration of luciferase when 0.01 mL of 1 × $10^{-6}$ M ATP is added to 0.35 mL of the luminescence reagent.

[Figure 19] Figure 19 illustrates the stability (change of luminescence level over time) of a luminescence reagent containing each concentration of luciferase when 0.01 mL of 1 × $10^{-7}$ M ATP is added to 0.35 mL of the luminescence reagent.

[Figure 20] Figure 20 illustrates the level of luminescence, after 1 hour, of the luminescence reagent containing each concentration of luciferase when 0.01 mL of 1 × $10^{-7}$ M ATP is added to 0.35 mL of the luminescence reagent.

[Figure 21] Figure 21 illustrates the level of luminescence when each component is excluded from a luminescence reagent and the excluded component is "mixed and then stored" or "stored and then mixed", followed by the addition of 0.1 mL of a 1 × $10^{-6}$ M ATP solution. The figure also illustrates the level of luminescence when 0.1 mL of 1 × $10^{-6}$ M ATP is added without storage (before storage).

Description

[Liquid composition]

The present disclosure describes the use of a liquid composition for measuring ATP and AMP and/or ADP in a sample after storage of the liquid composition, wherein

(i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
(ii) the relative luminescence level of the liquid composition during storage is 5500 RLU or less, and the relative luminescence level is a value determined by subtracting a control value from a measurement value.

The present disclosure further describes the use of a liquid composition for measuring ATP and AMP and/or ADP in a sample after storage of the liquid composition, wherein

(i) the liquid composition comprises luciferase, luciferin, an enzyme that catalyzes a reaction that produces ADP from AMP, a substrate of the enzyme that catalyzes a reaction that produces ADP from AMP, an enzyme that catalyzes a reaction that produces ATP from ADP, a substrate of the enzyme that catalyzes a reaction that produces ATP from ADP, and a cofactor, or when at least one of these components is not contained in the liquid composition, the component that is not contained in the liquid composition is added thereto before or during measurement, and
(ii) the relative luminescence level of the liquid composition during storage is 5500 RLU or less, and the relative luminescence level is a value determined by subtracting a control value from a measurement value.

In the compositions of the first and second instances, the measurement value is a value obtained by adding 0.35 mL of the liquid composition to a measurement tube of LuciPac Pen (manufactured by Kikkoman Biochemifa Company), then adding 0.01 mL of 1 × $10^{-7}$ M ATP (manufactured by Oriental Yeast Co., Ltd.) solution thereto, and leaving the mixture to stand at 25°C for 1 hour, followed by measurement using Lumitester Smart (manufactured by Kikkoman Biochemifa

Company), and the control value is a value obtained by measuring the level of luminescence under the same conditions as those for obtaining the measurement value except that sterile ultra pure water is added instead of the ATP solution. The measurement tube of LuciPac Pen (manufactured by Kikkoman Biochemifa Company) contains a luminescence reagent and is therefore used after this reagent is washed off to such an extent that the reagent does not influence the measurement system. If the measurement tube of LuciPac Pen (manufactured by Kikkoman Biochemifa Company) is difficult to obtain, an equivalent measurement tube of LuciPac A3 (manufactured by Kikkoman Biochemifa Company) may be used. As for the ATP, as a rule, ATP manufactured by Oriental Yeast Co., Ltd. is used. However, if this ATP is difficult to obtain or the like, then equivalent ATP can be used. It is preferable for the sterile ultra pure water to be less contaminated with ATP and ADP and AMP in order to avoid increasing the background value and influencing the measurement value. The sterile ultra pure water can be water having a total concentration of ATP and ADP and AMP of $1 \times 10^{-9}$ M or lower or $1 \times 10^{-10}$ M or lower. Sterile ultra pure water is used in the dilution of ATP, and the same sterile ultra pure water as that for background measurement is to be used.

When the total amount of the liquid composition (for example, the liquid composition in one kit) is less than 0.35 mL in measuring the relative luminescence level of the liquid composition during storage, a plurality of equivalent liquid compositions (for example, different liquid compositions in the same types of kits) may be mixed and the relative luminescence level of the liquid composition mixture can be measured.

As used herein, the relative luminescence level (relative level of luminescence) is a value obtained by measuring levels of luminescence under the same conditions as a rule. If the LuciPac Pen, for example, is difficult to obtain, the following conditions can be used instead: the measurement value is obtained by dividing, by 400, a value obtained by adding 0.35 mL of the liquid composition to Lumitube (manufactured by Kikkoman Biochemifa Company, 12ϕ × 54 mm), then adding 0.01 mL of a $1 \times 10^{-7}$ M ATP solution thereto, and leaving the mixture to stand at 25°C for 1 hour, followed by measuring with Lumitester C-110 (manufactured by Kikkoman Biochemifa Company); and the control value is obtained by dividing, by 400, a value obtained by measuring the level of luminescence under the same conditions as those for obtaining the measurement value except that sterile ultra pure water is added instead of the ATP solution.

In one instance, the relative luminescence level of the liquid composition during storage may be 5500 RLU or less, 5000 RLU or less, 4500 RLU or less, 4000 RLU or less, 3500 RLU or less, or 3000 RLU or less. The relative luminescence level of the liquid composition during storage can be, for example, 2900 RLU or less, 2800 RLU or less, 2700 RLU or less, 2600 RLU or less, 2500 RLU or less, 2400 RLU or less, 2300 RLU or less, 2200 RLU or less, 2100 RLU or less, 2000 RLU or less, 1900 RLU or less, 1800 RLU or less, 1700 RLU or less, 1600 RLU or less, 1500 RLU or less, 1400 RLU or less, 1300 RLU or less, 1200 RLU or less, 1100 RLU or less, 1000 RLU or less, 900 RLU or less, 800 RLU or less, 700 RLU or less, 600 RLU or less, 500 RLU or less, 400 RLU or less, 300 RLU or less, 200 RLU or less, or 100 RLU or less. In the Examples, it is shown that a lower relative luminescence level of the liquid composition during storage increases the stability of the liquid composition. When the relative luminescence level of the liquid composition during storage is 2500 RLU or less or 2300 RLU or less, the liquid composition can be particularly excellent in stability. As used herein, the term "excellent in stability" of the liquid composition means, for example, decrease in luminescence level of the liquid composition is small or absent.

Examples of methods for decreasing the relative luminescence level of the liquid composition during storage include, but are not limited to, a method of changing storage pH from the optimum pH of an enzyme, a method of degrading ATP by the addition of ATPase or the like so as not to emit luminescence, a method of adding a reaction inhibitor, and a method of lowering the concentration of a component necessary for cycling reaction, or excluding the component and then adding the excluded component before measurement or during measurement, and combinations of these methods.

For example, in the method of changing storage pH from the optimum pH, the pH of the liquid composition may be elevated or lowered from the optimum pH to the extent that enzymatic reaction does not progress or is delayed and that an enzyme is not denatured, and the pH of the liquid composition can be re-adjusted to the optimum pH before or during measurement. In the case of rendering the composition acidic, storage pH can be, for example, 6 or lower, 5 or lower, or 4 or lower and can be 2 or higher or 3 or higher. In the case of rendering the composition basic, storage pH can be, for example, 9 or higher, 10 or higher, or 11 or higher and can be 13 or lower or 12 or lower. Those skilled in the art can readily determine the pH that decreases the relative luminescence level of the liquid composition without denaturing an enzyme, and can also readily adjust the pH to the determined pH using an acid or a base. For example, as for HLK described in JP Patent Publication (Kokai) No. 11-239493 A (1999), the relative luminescence level can be decreased to approximately 1/3 or lower by adjusting pH to 6.5 or lower and can be decreased to approximately 1/10 or lower by adjusting pH to 6 or lower. The pH before or during measurement can be 6 to 9, 7.5 to 8.5, or approximately 8. In another instance, storage pH and the pH before or during measurement are the same or about the same (rarely different) (for example, the difference between storage pH and the pH before or during measurement can be within 1).

Examples of the method for degrading ATP by the addition of ATPase include a method of adding adenosine phosphate deaminase during storage. The ATPase can be used at a concentration that does not influence the ATP measurement system, and the influence of the ATPase may be reduced by diluting the liquid composition before or during measurement. Those skilled in the art can readily determine the concentration and type of the ATPase.

In the case of adding a reaction inhibitor, examples of the reaction inhibitor include metal salts such as NaCl and surfactants such as benzalkonium chloride. The influence of the reaction inhibitor may be reduced by diluting the liquid composition before or during measurement. Alternatively, the reaction inhibitor may be removed before or during measurement. For example, a metal salt can be removed using a chelating agent, and benzalkonium chloride can be removed using cyclodextrin. Those skilled in the art can readily determine the concentration and type of the reaction inhibitor.

The method of lowering the concentration of a component necessary for cycling reaction, or excluding the component and then adding the excluded component before or during measurement is as described herein.

In the first aspect, the present invention relates to the use of a liquid composition for measuring ATP and AMP and/or ADP in a sample after storage of the liquid composition, wherein the liquid composition has been stored one day or longer, and wherein

(i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
(ii) at least one or more of the following conditions are satisfied:

the concentration of luciferin in the liquid composition is 0.4 mM or lower;
the concentration of luciferase in the liquid composition based on the Bradford method is 0.3 mg/mL or lower;
the concentration of the enzyme that catalyzes a reaction that produces ATP from AMP (for example, PPDK) in the liquid composition is 1 U/mL or lower;
the concentration of the substrate of the enzyme that catalyzes a reaction that produces ATP from AMP (for example, pyrophosphoric acid or a salt thereof and/or phosphoenolpyruvic acid or a salt thereof when the enzyme is PPDK) in the liquid composition is 0.1 mM or lower; and
the concentration of the cofactor (for example, magnesium salt) in the liquid composition is 6 mM or lower.

In the second aspect, the present invention relates to the use of a liquid composition for measuring ATP and AMP and/or ADP in a sample after storage of the liquid composition, wherein the liquid composition has been stored 1 day or longer, and wherein

(i) the liquid composition comprises luciferase, luciferin, an enzyme that catalyzes a reaction that produces ADP from AMP, a substrate of the enzyme that catalyzes a reaction that produces ADP from AMP, an enzyme that catalyzes a reaction that produces ATP from ADP, a substrate of the enzyme that catalyzes a reaction that produces ATP from ADP, and a cofactor, or when at least one of these components is not contained in the liquid composition, the component that is not contained in the liquid composition is added thereto before or during measurement, and
(ii) at least one or more of the following conditions are satisfied:

the concentration of the enzyme that catalyzes a reaction that produces ADP from AMP (for example, ADK) in the liquid composition is 450 U/mL or lower;
the concentration of the substrate of the enzyme that catalyzes a reaction that produces ADP from AMP in the liquid composition is 0.1 mM or lower (for example, the substrate is unnecessary for ADK);
the concentration of the enzyme that catalyzes a reaction that produces ATP from ADP (for example, PK) in the liquid composition is 20 U/mL or lower;
the concentration of the substrate of the enzyme that catalyzes a reaction that produces ATP from ADP (for example, phosphoenolpyruvic acid or a salt thereof when the enzyme is PK) is 1.2 mM or lower; and
the concentration of the cofactor (for example, magnesium salt) in the liquid composition is 6 mM or lower.

In one embodiment, the liquid composition of the first aspect further comprises at least one component selected from an enzyme that catalyzes a reaction that produces ATP from ADP, a substrate of the enzyme that catalyzes a reaction that produces ATP from ADP, an enzyme that catalyzes a reaction that produces AMP from ADP, and a substrate of the enzyme that catalyzes a reaction that produces AMP from ADP, or at least one of these components is added to the liquid composition before or during measurement.

Hereinafter, the liquid compositions of the first to second aspects (in the present specification, these are also collectively referred to as the "liquid composition described herein"), and components constituting the same, etc. will be described in more detail.

In one embodiment, the liquid composition described herein is directed to measuring ATP as well as AMP and/or ADP (i.e., ATP and ADP; ATP and AMP; or ATP, ADP and AMP). Biogenic substances contain ATP. While ATP can be

relatively easily dephosphorylated into ADP, and ADP can also be dephosphorylated into AMP in some cases. Thus, the measurement of the 2 components ATP and ADP or AMP or the 3 components ATP, ADP, and AMP allows for the stable measurement of ATP (or degradation products thereof) contained in a biogenic substance. Thus, the measurement of the 2 components ATP and ADP or AMP or the 3 components ATP, ADP, and AMP allows for more accurate determination of cleanliness without missing uncleanness.

[Luciferase]

Luciferase is a generic name for oxidase that brings about bioluminescence. In one embodiment, the luciferase catalyzes a reaction that converts ATP, $O_2$, and luciferin into AMP, pyrophosphoric acid, $CO_2$, and oxyluciferin, and luminescence is generated during this reaction. The luciferase may be a natural luciferase or may be a genetically engineered recombinant luciferase mutant. The luciferase mutant may be generated by site-directed mutagenesis or by random mutagenesis. The luciferase mutant may be a fusion protein with a protein having another function. The luciferase mutant may have desired properties such as improved heat resistance, or improved surfactant resistance.

The level of luminescence from luciferase can be evaluated using relative luminescence intensity (RLU) as an indicator obtained using an appropriate apparatus for measuring luminescence, for example, a luminometer (Lumitester Smart, Lumitester PD-20, or Lumitester PD-30 manufactured by Kikkoman Biochemifa Company, or the like) or an apparatus with a photodiode (SystemSURE Plus or EnSURE manufactured by Hygiena, LLC, AccuPoint Advanced manufactured by Neogen Corporation, or the like). Typically, luminescence generated during the conversion of luciferin into oxyluciferin is measured. As apparatuses for measuring luminescence, apparatuses with a photomultiplier tube (Clean Trace LM1 or Clean Trace UNG3 manufactured by 3M Corporation, Lumitester C-110 or Lumitester C-100 manufactured by Kikkoman Biochemifa Company, Junior LB9509, CentroLB960 or Lumat3 LB9508 manufactured by Berthold Technologies GmbH & Co. KG, and the like) capable of high sensitivity measurement can also be used. Apparatuses capable of high sensitivity measurement are useful for accurate measurement, particularly when the level of luminescence is decreased.

The luciferase is not particularly limited, as long as its substrate is ATP, and those derived from bacteria, protozoans, animals, mollusks, and insects can be used. Examples of those derived from insects include coleopteran luciferase, and examples thereof include those derived from fireflies such as the genus Photinus, for example, Photinus pyralis; the genus Photuris, for example, Photuris lucicrescens, Photuris pennsylvanica; the genus Luciola, for example, Luciola cruciata, Luciola lateralis, Luciola parvula; the genus Pyrocoelia; and Lucidina biplagiata; and those derived from click beetle in the genus Pyrophorus. Many luciferase genes have been reported, and their nucleotide sequences and amino acid sequences are available from known databases such as GeneBank.

The luciferase gene may be a wildtype gene or a gene having a mutation. The mutation may be a mutation introduced site-specifically or a random mutation. Examples of known mutations include, but are not limited to, mutations that improve the level of luminescence as described in JP Patent Publication (Kokai) No. 2011-188787 A; mutations that increase the luminescence durability as described in JP Patent Publication (Kokai) No. 2000-197484 A; mutations that change the luminescence wavelength as described in JP Patent No. 2666561 or JP Patent Publication (Kohyo) No. 2003-512071 A; mutations that increase resistance to surfactant as described in JP Patent Publication (Kokai) No. 11-239493 A (1999); mutations that increase affinity for substrate as described in International Publication No. WO 99/02697 pamphlet, JP Patent Publication (Kohyo) No. 10-512750 A (1998), or JP Patent Publication (Kohyo) No. 2001-518799 A; and mutations that increase the stability as described in JP Patent No. 3048466, JP Patent Publication (Kokai) No. 2000-197487 A, JP Patent Publication (Kohyo) No. 9-510610 A (1997), and JP Patent Publication (Kohyo) No. 2003-518912 A.

The luciferase gene and a recombinant DNA thereof can be prepared in a conventional method. For example, JP Patent Publication (Kokoku) No. 7-112434 B (1995) describes a luciferase gene from Luciola lateralis. Moreover, JP Patent Publication (Kokai) No. 1-51086 A (1989) describes a luciferase gene from Luciola cruciata.

The luciferase gene can be incorporated into a vector such as a plasmid, a bacteriophage, or a cosmid, with which an appropriate host may be transformed or transfected. The host may be a microorganism, a bacterium such as Escherichia coli, yeast, or the like. The transformed host having luciferase-producing ability can be cultured by various known methods.

Examples of the medium include those obtained by adding, to one or more nitrogen sources such as triptone, yeast extract, meat extract, peptone, corn steep liquor, or an exudate of soybean or wheat bran, one or more inorganic salts such as sodium chloride, potassium dihydrogen phosphate, dipotassium phosphate, magnesium chloride, ferric chloride, magnesium sulfate, or manganese sulfate, and as necessary carbohydrate raw materials, vitamins, and the like.

The initial pH of the medium can be, for example, 7 to 9. The culture can be conducted, for example, at 30 to 40°C for 2 to 24 hours, by aerated and agitated culture, shaking culture, static culture, or the like. After culturing, the luciferase is collected from the culture by a known technique.

Specifically, luciferase is extracted by subjecting the cells to an ultrasonic homogenization treatment, a grinding

treatment, or the like by a conventional method or using a lytic enzyme such as lysozyme. A crude enzyme can be obtained by treating the resultant extract by filtration, centrifugation, or the like, removing nucleic acid by streptomycin sulfate or the like as needed, and adding ammonium sulfate, alcohol, acetone, or the like to fractionate the same.

The crude enzyme may further be purified by various techniques such as gel filtration and chromatography. Commercially available luciferases can also be used and, for example, the luciferase of Kikkoman Biochemifa Company, catalogue No. 61314 can be used. This luciferase is the luciferase described in JP Patent Publication (Kokai) No. 11-239493 A (1999) (JP Patent No. 3749628) (SEQ ID NO: 1). Moreover, a commercially available luciferase from Sigma Aldrich, Promega KK., or Molecular Probes (R) of Life Technology Inc. can also be used.

In one embodiment, the luciferase concentration of the liquid composition described herein can be a concentration of, for example, 0.3 mg/mL or lower, 0.25 mg/mL or lower, 0.2 mg/mL or lower, 0.1 mg/mL or lower, 0.05 mg/mL or lower, 0.01 mg/mL or lower, 0.005 mg/mL or lower, 0.001 mg/mL or lower, or 0.0005 mg/mL or lower based on the Bradford method. The concentration based on the Bradford method can be measured using a BSA solution as a standard and Coomassie (Bradford) Protein Assay Kit (manufactured by Thermo Fisher Scientific, Inc.), as described in the Examples. If this kit, for example, is difficult to obtain, the luciferase concentration can be measured by the Bradford method known to those skilled in the art using the same BSA solution as above as a standard such that an equivalent value is obtained. Alternatively, the concentration based on the Bradford method can also be measured by determining absorbance according to a 280 nm absorbance method, followed by conversion, as described in the Examples. While a lower luciferase concentration is capable of improving the stability of the liquid composition, the level of luminescence as a whole decreases. Therefore, if the level of luminescence is too low, luciferase may be added before or during measurement. The luciferase concentration of the liquid composition during storage or the liquid composition supplemented with luciferase before or during measurement can be, for example, 0.00001 mg/mL or higher, 0.0001 mg/mL or higher, 0.001 mg/mL or higher, or 0.01 mg/mL or higher in terms of the concentration based on the Bradford method.

In the present specification, the luciferase concentration is a value based on the Bradford method as a rule. However, when the liquid composition is prepared so as to contain a protein other than luciferase, the concentration of luciferase alone in the liquid composition thus prepared cannot be directly measured by the Bradford method. In this case, the luciferase concentration based on the Bradford method can be indirectly measured on the basis of the activity of luciferase as described below.

The liquid composition containing luciferase is diluted with an enzyme diluent (5.0% glycerol, 1.0 mM EDTA.2Na.2$H_2O$, 1.0 mM 2-mercaptoethanol, 50 mM tricine, 1.0% bovine serum albumin (BSA) (pH 7.8)) such that the level of luminescence becomes 100,000 to 1,000,000 RLU. A 100 μL aliquot of the liquid composition diluted with the enzyme diluent is collected into a Rohren tube (manufactured by Sarstedt K.K.) prewarmed to 25°C, to which 100 μL of a luminescence reagent for luciferase activity measurement (50 mM tricine, 4.0 mM ATP·2Na, 2.0 mM D-luciferin, 10 mM $MgSO_4 \cdot 7H_2O$ (pH 7.8)) is then added using an injector. The level of luminescence is measured for 20 seconds from 0.5 seconds later using LUMAT LB9507 (manufactured by Berthold Technologies GmbH & Co. KG), and the level of luminescence obtained here is regarded as a sample luminescence level (Es). The measurement is performed at 25°C. Likewise, a blank luminescence level (E0) is measured using the enzyme diluent instead of the luciferase solution.

Luciferase activity (LU/mL) = (Es - E0) × Dilution ratio / Amount of the sample solution Amount of the sample solution: 0.1 mL

In addition to the experiment described above, luciferase confirmed to have a single band in SDS-PAGE was used to measure the activity value and protein concentration of the luciferase. As a result, the luciferase activity was $8.4 \times 10^{14}$ LU/mL, while the value based on the Bradford method was 39.4 mg/mL. That is, the specific activity is $2.1 \times 10^{13}$ LU/mg. Luciferase activity can be converted to protein concentration by use of the numeric value of this specific activity. That is,

Protein concentration based on the Bradford method (mg/mL) = Luciferase activity (LU/mL) / ($2.1 \times 10^{13}$ LU/mg)

This enables the protein concentration of luciferase to be determined even for reagents containing a protein as a stabilizing agent such as BSA or an enzyme for cycling (for example, PPDK or PK). While this converted value is a value obtained for a particular luciferase (HLK described in JP Patent Publication (Kokai) No. 11-239493 A (1999)), in the case of using a different luciferase, such converted value can be calculated in the same manner as above and the concentration of the luciferase can be measured based on the same.

When the liquid composition contains a protein other than luciferase, the luciferase concentration can also be measured, aside from the activity measurement, by fractionating the liquid composition by chromatography such as HPLC, and measuring the protein level of the luciferase fraction by the Bradford method.

[Luciferin]

The luciferin may be any luciferin as long as it is recognized as a substrate by the luciferase being used and may be natural or chemically synthesized. Moreover, known luciferin derivatives may also be used. The basic structure of luciferin is imidazopyrazinone, and there are many tautomers thereof. Examples of luciferin include firefly luciferin. Firefly luciferin is a substrate of the firefly luciferase (EC 1.13.12.7). Luciferin derivatives may be those described in JP Patent Publication (Kokai) No. 2007-91695 A, or JP Patent Publication (Kohyo) No. 2010-523149 A (International Publication No. 2008/127677).

In one embodiment, the concentration of luciferin or a derivative thereof in the liquid composition described herein can be, for example, 0.4 mM or lower, 0.35 mM or lower, 0.3 mM or lower, 0.25 mM or lower, 0.15 mM or lower, 0.1 mM or lower, 0.05 mM or lower, 0.02 mM or lower, 0.01 mM or lower, 0.005 mM or lower, 0.001 mM or lower, or 0.0001 mM or lower. While a lower luciferin concentration is capable of improving the stability of the liquid composition, the level of luminescence as a whole decreases. Therefore, if the level of luminescence is too low, luciferin or a derivative thereof may be added before or during measurement. The concentration of luciferin or a derivative thereof in the liquid composition during storage or the liquid composition supplemented with luciferin before or during measurement can be, for example, 0.00001 mM or higher, 0.0001 mM or higher, 0.001 mM or higher, or 0.01 mM or higher.

The concentration of luciferin or luciferin derivative can be measured on the basis of the level of luminescence in accordance with the methods described above about luciferase. The concentration of luciferin can be measured, for example, by diluting a luminescence reagent without influencing other components such as enzymes, then preparing reagents containing different concentrations of luciferin in the presence of excess luciferase, and comparing their levels of luminescence with those of standard materials containing known concentrations of luciferin. Alternatively, the concentration of luciferin can also be measured by fractionating the liquid composition by chromatography such as HPLC, detecting a peak corresponding to luciferin, and comparing the peak intensity with that of standard materials containing known concentrations of luciferin.

It was unexpected that a lower luciferin concentration was capable of improving the stability of the liquid composition, as described below. In an ordinary measurement system using enzymatic reaction, the substrate is added at a high concentration such that the decreased level of the substrate with the progression of the reaction does not influence the reaction rate. In particular, as for cycling reactions, it is believed to be reasonable to those skilled in the art that since the substrate is continuously consumed, designing the substrate concentration at a higher concentration will lead to the durability of the luminescence level of the reagent and its stabilization during storage. However, results of the Examples described herein have revealed that in a cycling luminescence reagent that utilizes a luciferin-luciferase reaction, a low concentration of luciferin can rather stabilize the level of luminescence of the cycling luminescence reagent. Without wishing to be bound by any theory, decrease in the amount of luciferin ascribable to the consumption of the luciferin may not be the principal cause of reduction in the stability of the luminescence level caused by the storage of a cycling luminescence reagent in a liquid state, and the inhibition of luminescence by oxyluciferin produced through the luminescent reaction may be the principal cause thereof. In other words, this may be different from phenomena that occur in cycling methods using other enzymes and may be a phenomenon unique to a liquid cycling luminescence reagent that utilizes a luciferin-luciferase reaction.

[An enzyme that catalyzes a reaction that produces ATP from AMP]

In one embodiment, the liquid composition described herein comprises an enzyme that catalyzes a reaction that produces ATP from AMP. By the enzyme that catalyzes a reaction that produces ATP from AMP, the AMP present in the system is converted into ATP. Then, ATP is converted into AMP by luciferase and luminescence is produced along with the conversion. Thus, in the present embodiment, in addition to ATP, AMP can also be measured.

As the enzyme that catalyzes a reaction that produces ATP from AMP, a known enzyme can be used. Examples thereof include, but are not limited to, pyruvate-phosphate dikinase (PPDK), pyruvate-water dikinase (PWDK), and combinations thereof.

[Pyruvate-phosphate dikinase (PPDK)]

Pyruvate-phosphate dikinase (EC 2.7.9.1) catalyzes the reaction between ATP, pyruvate, and orthophosphate, and adenosine monophosphate (AMP), phosphoenolpyruvate (PEP), and pyrophosphate (PPi):

$$\text{ATP + pyruvate + phosphate} \longleftrightarrow \text{AMP + PEP + PPi}$$

Pyruvate-phosphate dikinase (PPDK) is also referred to as ATP:pyruvate, phosphate phosphotransferase, pyruvate orthophosphate dikinase, pyruvate phosphate ligase. As used herein, these terms are mutually exchangeable.

Usually, PPDK converts pyruvate into PEP, and 1 molecule of ATP is consumed and converted into AMP in the process. The reaction is divided into the following 3 reversible reactions.

1. PPDK binds to ATP and produces AMP and diphosphorylated PPDK.
2. The diphosphorylated PPDK binds to inorganic phosphate and produces diphosphate and monophosphorylated PPDK.
3. The monophosphorylated PPDK binds to pyruvate and produces PEP and also produces PPDK.

In the reactions, if the concentration of PEP present in the system is high, reactions progress in the reverse direction as follows.

[Formula 1]

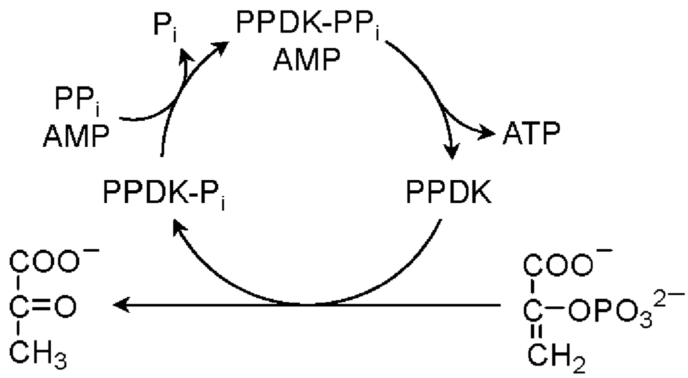


For convenience, the reaction steps are described with the same number as those described above.

3. **PEP binds** to PPDK and produces monophosphorylated PPDK and pyruvate.
2. From the diphosphate and monophosphorylated PPDK, diphosphorylated PPDK and inorganic phosphate are produced.
1. From the diphosphorylated PPDK and AMP, PPDK and ATP are produced.

PPDK is not particularly limited, and examples thereof include those derived from microorganisms such as Microbispora thermorosea described in JP Patent Publication (Kokai) No. 8-168375 A (1996), Propionibacterium shremanii, Bacteroides symbiosus, Entamoeba histolytica, Acetobacter xylinum, and Propionibacter shermanii, and those derived from plants such as corn and sugarcane.

[Pyruvate-water dikinase (PWDK)]

Pyruvate-water dikinase (EC 2.7.9.2) catalyzes the following reaction:

$$\text{ATP + pyruvate + } H_2O \longleftrightarrow \text{AMP + phosphoenolpyruvate (PEP) + phosphate (P)}$$

Pyruvate-water dikinase is also referred to as phosphoenolpyruvate synthase; pyruvate-water dikinase (phosphorylation); PEP synthetase; phosphoenolpyruvate synthetase; phosphoenolpyruvic synthetase; and phosphopyruvate synthetase. As used herein, these terms are mutually exchangeable.

PWDK is not particularly limited, and examples thereof include those derived from Escherichia coli, Pseudomonas fluorescens, Pyrococcus furiosus, Staphylothermus marinus, Sulfolobus solfataricus, Thermococcus kodakarensis, Thermoproteus tenax, and corn (Zea mays).

By using PWDK in combination with PEP, the ATP production from AMP and PEP can be promoted. By combining an enzyme that catalyzes a reaction that produces ATP from AMP with an enzyme that catalyzes a reaction that produces ATP from ADP, described herein, ADP is converted into ATP, and as a result, ATP and ADP and AMP can be measured.

[An enzyme that catalyzes a reaction that produces ATP from ADP]

In one embodiment, the liquid composition described herein comprises an enzyme that catalyzes a reaction that produces ATP from ADP. Due to the enzyme that catalyzes a reaction that produces ATP from ADP, the ADP present in the

system is converted into ATP. Then, ATP is converted into AMP by luciferase and luminescence is produced along with the conversion. Thus, in the present embodiment, ATP as well as ADP can be measured.

As the enzyme that catalyzes a reaction that produces ATP from ADP, known enzymes can be used, and examples thereof include kinases having ATP-producing ability. Examples of kinases having ATP-producing ability include, but are not limited to, pyruvate kinase, acetate kinase, creatine kinase, polyphosphate kinase, riboflavin kinase, phosphofructokinase, fructose-bisphosphatase, hexokinase, glucokinase, glycerol kinase, fructokinase, and combinations thereof.

[Pyruvate kinase (PK)]

Pyruvate kinase (EC 2.7.1.40) converts phosphoenolpyruvate into pyruvate in glycolysis, and ADP is converted into ATP during the conversion. This reaction is an exergonic reaction, where the change in the Gibbs energy is negative, and irreversible under natural conditions: PEP + ADP → pyruvate + ATP

The reverse reaction is catalyzed by pyruvate carboxylase and phosphoenolpyruvate carboxykinase in gluconeogenesis and produces PEP and ADP from ATP and pyruvic acid. When cell extraction is performed, various enzymes coexist in the system and the aforementioned reactions can progress in both directions. Under such conditions, if phosphoenolpyruvate is present in the system at a high concentration, ADP can be converted into ATP. Moreover, if not only phosphoenolpyruvate, but also pyruvate kinase is present in the system, then it is believed that more ADP can be converted into ATP. PK is not particularly limited, and, for example, those derived from animals such as rabbit, rat, and chicken and microorganisms such as yeast and Bacillus stearothermophilus can be used.

[Acetate kinase (AK)]

Acetate kinase (EC 2.7.2.1) catalyzes conversion from ATP and acetate to ADP and acetylated phosphate and vice versa in the presence of a cation:

ATP + acetate ←→ ADP + acetylated phosphate

Acetate kinase (AK) is also referred to as ATP:acetate phosphotransferase or acetyl kinase. As used herein, these terms are mutually exchangeable. In organisms *(in vivo),* ADP and acetylated phosphate are produced from ATP and acetate, and ultimately, reactions to produce acetyl CoA are promoted. If acetylated phosphate and ADP produced from acetyl CoA are present in the system, these can be converted into acetate and ATP. AK is not particularly limited, and those derived from microorganisms such as Escherichia coli, Bacillus stearothermophilus, Costridium pasteurianum, Lactobacillus delbruckii, and Veillonella alcalescence can be used.

[Creatine kinase (CK)]

Creatine kinase (EC 2.7.3.2) mediates the conversion reaction from creatine and ATP to creatine phosphate and ADP and vice versa:

Creatine + ATP ←→ creatine phosphate + ADP

Creatine kinase (CK) is also referred to as creatine phosphokinase (CPK) or phosphocreatine kinase. As used herein, these terms are mutually exchangeable. In muscles and the like of animals, creatine phosphate and ADP are usually produced from creatine and ATP. However, this reaction is a reversible reaction, and if creatine phosphate and ADP are present in the system at high concentrations, the reaction may progress in the reverse direction to produce creatine and ATP. In organisms, cytoplasmic CK is composed of two subunits of B or M. Therefore, the 3 isozymes CK-MM, CK-BB, and CK-MB can be present depending on the combination of the subunits. The isozymic pattern differs depending on the tissue, but any combination can be used in the present invention. CK is not particularly limited, and those derived from animals can be used, and examples thereof include those derived from rabbit, chicken, cow, pig, carp, catfish, and frog.

[Polyphosphate kinase (PPK)]

Polyphosphate kinase (EC 2.7.4.1) catalyzes the reaction to convert polyphosphate (PolyPn) and ADP into polyphosphate (PolyPn-1) and ATP:

ADP + PolyPn ←→ ATP + PolyPn-1

Polyphosphate kinase (PPK) is also referred to as ATP:polyphosphate phosphotransferase. As used herein, these terms are mutually exchangeable. PPK is involved in oxidative phosphorylation in organisms. If polyphosphate (n) and ADP are present in the system, these can be converted into polyphosphate (n-1) and ATP. The PPK is not particularly limited, but, for example, those derived from microorganisms such as Escherichia coli, yeast, and Corynebacterium xerosis can be used.

[Riboflavin kinase (FMNK)]

Riboflavin kinase (EC 2.7.1.26) is also described as FMNK and catalyzes the reaction to convert riboflavin and ATP into riboflavin phosphate (FMN) and ADP:

$$\text{ATP + riboflavin} \longleftrightarrow \text{ADP + FMN}$$

Riboflavin kinase belongs to ATP:riboflavin 5'-phosphotransferase (also referred to as flavokinase). FMNK is not particularly limited, and, for example, those derived from microorganisms and animals can be used, and examples thereof include those derived from yeast, rat, and a bean (Phaseolus radiatus).

[Phosphofructokinase 1 (PFK1)]

Phosphofructokinase 1 (EC 2.7.1.11) is also described as PFK1 and catalyzes the reaction to convert fructose-6-phosphate (Fru6P) and ATP into fructose-1,6-bisphosphate (Fru1,6-BP) and ADP:

$$\text{Fru6P + ATP} \longleftrightarrow \text{Fru1,6-BP + ADP}$$

Phosphofructokinase 1 belongs to phosphofructokinase. As used herein, phosphofructokinase 1 may be described as Fru-1,6BPK. PFK1 is not particularly limited, and those derived from animals and microorganisms can be used, and examples of those derived from microorganisms include those derived from baker's yeast, brewer's yeast, Clostridium pasteurianum, Escherichia coli, and Bacillus licheniformis.

[Fructose-bisphosphatase (FBPase)]

Fructose-bisphosphatase (EC 3.1.3.11) is also described as FBPase and catalyzes the reaction to convert fructose-1,6-bisphosphate (Fru1,6-BP) and ADP into fructose-6-phosphate (Fru6P) and ATP:

$$\text{Fru1,6-BP + ADP} \longleftrightarrow \text{Fru6P + ATP}$$

FBPase may also be described as FBP, FBP1. FBPase is not particularly limited, and those derived from animals, plants, and microorganisms can be used, and examples thereof include those derived from rabbit and chicken.

[An enzyme that catalyzes a reaction that produces AMP from ADP]

In one embodiment, the liquid composition described herein comprises an enzyme that catalyzes a reaction that produces AMP from ADP. Due to the enzyme that catalyzes a reaction that produces AMP from ADP, the ADP present in the system is converted into AMP. By combining the enzyme that produces AMP from ADP and the enzyme that produces ATP from AMP (for example, PPDK), ADP is converted into AMP and AMP is converted into ATP, and as a result, ATP and ADP and AMP can be measured.

As the enzyme that catalyzes a reaction that produces AMP from ADP, known enzymes can be used. Examples thereof include, but are not limited to, ADP-dependent hexokinase, apyrase, and combinations thereof.

[ADP-dependent hexokinase]

ADP-dependent hexokinase (EC 2.7.1.147) is also referred to as ADP-specific hexokinase and catalyzes the following reaction:

$$\text{D-glucose + ADP} \longleftrightarrow \text{D-glucose-6-phosphate + AMP}$$

[Apyrase]

Apyrase (EC 3.6.1.5) is also referred to as adenosine diphosphatase, ADPase, ATP diphosphatase, or ATP diphosphohydrolase and catalyzes the following two reactions:

$$ATP + H_2O \rightarrow ADP + phosphate\ (P)$$

$$ADP + H_2O \rightarrow AMP + phosphate\ (P)$$

[An enzyme that catalyzes a reaction that produces ADP from AMP]

In one embodiment, the liquid composition described herein comprises an enzyme that catalyzes a reaction that produces ADP from AMP. Due to the enzyme that catalyzes a reaction that produces ADP from AMP, the AMP present in the system is converted into ADP. By combining the enzyme that produces ADP from AMP and the enzyme that produces ATP from ADP (for example, PK), AMP is converted into ADP and ADP is converted into ATP, and as a result, ATP and ADP and AMP can be measured.

As the enzyme that catalyzes a reaction that produces ADP from AMP, known enzymes can be used. Examples thereof include, but are not limited to, adenylate kinase (ADK).

[Adenylate kinase (ADK)]

Adenylate kinase (EC 2.7.4.3) is also referred to as ADK and catalyzes the following reaction in the presence of a metal ion:

$$ATP + AMP \longleftrightarrow 2ADP$$

This reaction is reversible. ADK is an example of an enzyme that catalyzes the reaction that produces ADP from AMP.

ADK is not particularly limited, and examples thereof include those derived from microorganisms such as yeast and those derived from animals such as rabbit, pig, cow, rat, and pig.

[RNase]

In one embodiment, the method of the present invention may comprise using an RNase. The RNase here means an RNase not from the sample (non-intrinsic RNase).

In one embodiment, the liquid composition described herein may comprise an RNase. As used herein, RNase means an RNase not from the sample (non-intrinsic RNase). By using the RNase, RNA is degraded into AMP and cleanliness can be measured broadly including RNA.

As used herein, RNase means an enzyme that catalyzes the reaction that produces 5'-mononucleotide (AMP, GMP, CMP, and UMP) from RNA, and examples thereof include the following: (1) Endonuclease $S_1$ (EC3.1.30.1), (2) Venom exonuclease (EC3.1.15.1), (3) Phosphodiesterase 1 (EC3.1.4.1). The Endonuclease $S_1$ includes Nuclease $P_1$, Mung beans nuclease, and Neurospora crassa nuclease.

As used herein, the enzyme that catalyzes a reaction that produces ATP from AMP (for example, PPDK or PWDK), the enzyme that catalyzes a reaction that produces ATP from ADP, the enzyme that catalyzes a reaction that produces AMP from ADP, and the enzyme that catalyzes a reaction that produces ADP from AMP (for example, ADK) described above may be collectively referred to as enzymes having ATP-producing ability.

The enzymes having ATP-producing ability that can be used include any known enzymes such as those derived from microorganisms, bacteria, eukaryotes, protists, plants, and animals, and, for example, a commercially available enzyme can be used. The amount of the enzyme added can be set as appropriate depending on the concentration and the reaction system of interest.

Various enzymes having ATP-producing ability are known. As used herein, the activity unit (U) of the enzymes having ATP-producing ability is defined as the amount of the enzyme that converts 1.0 μmol of substrate into ATP per minute at 37°C at pH 7.8, in view of the ATP-producing ability of the enzymes (1 U = 1 μmol ATP/min, pH 7.8, 37°C). Although the activity unit (U) of the enzymes having ATP-producing ability is defined as described above as a rule, the activity unit (U) of only the enzyme that catalyzes a reaction that produces ATP from ADP (for example, PK) is exceptionally defined as the amount of the enzyme that converts 1.0 μmol of substrate into ATP per minute at 25°C at pH 7.4 (1 U = 1 μmol ATP/min, pH 7.4, 25°C). In one embodiment, an enzyme having ATP-producing ability can be added such that the

activity unit in the system of measurement is 0.001 U or more, 0.01 U or more, 0.1 U or more, 1 U or more, 2 U or more, 3 U or more, 4 U or more, or 5 U or more. In one embodiment, an enzyme having ATP-producing ability can be added such that the activity unit in the system of measurement is 10000 U or less, 1000 U or less, 100 U or less, 50 U or less, 10 U or less, 9 U or less, 8 U or less, 7 U or less, or 6 U or less. A person skilled in the art can determine the amount of the enzyme added as appropriate. In one embodiment, the concentration of an enzyme having ATP-producing ability (or the respective concentrations or total concentration of a plurality of enzymes having ATP-producing ability; the same applies to description below) in the liquid composition described herein can be, for example, 20 U/mL or lower, 10 U/mL or lower, 5 U/mL or lower, 1 U/mL or lower, 0.5 U/mL or lower, 0.1 U/mL or lower, 0.05 U/mL or lower, 0.01 U/mL or lower, or 0.001 U/mL or lower. For example, the concentration of the enzyme that catalyzes a reaction that produces ATP from AMP (for example, PPDK) can be 1 U/mL or lower, 0.5 U/mL or lower, 0.1 U/mL or lower, 0.05 U/mL or lower, 0.01 U/mL or lower, or 0.001 U/mL or lower, and the concentration of the enzyme that catalyzes a reaction that produces ATP from ADP (for example, PK) can be 20 U/mL or lower, 10 U/mL or lower, 5 U/mL or lower, 1 U/mL or lower, 0.5 U/mL or lower, 0.1 U/mL or lower, 0.05 U/mL or lower, 0.01 U/mL or lower, or 0.001 U/mL or lower. While a lower concentration of the enzyme having ATP-producing ability is capable of improving the stability of the liquid composition, the level of luminescence as a whole decreases. Therefore, if the level of luminescence is too low, the enzyme having ATP-producing ability may be added before or during measurement. The concentration of the enzyme having ATP-producing ability (for example, the enzyme that catalyzes a reaction that produces ATP from AMP (for example, PPDK) and/or the enzyme that catalyzes a reaction that produces ATP from ADP (for example, PK)) in the liquid composition during storage or the liquid composition supplemented with the enzyme having ATP-producing ability before or during measurement can be, for example, 0.00001 U/mL or higher, 0.0001 U/mL or higher, 0.001 U/mL or higher, 0.01 U/mL or higher, 0.1 U/mL or higher, 1 U/mL or higher or 10 U/mL or higher.

When an enzyme having ATP-producing ability is used, the substrate of each enzyme (for example, a substrate of the enzyme that catalyzes a reaction that produces ATP from AMP, a substrate of the enzyme that catalyzes a reaction that produces ATP from ADP, a substrate of the enzyme that catalyzes a reaction that produces AMP from ADP, or a substrate of the enzyme that catalyzes a reaction that produces ADP or ATP from AMP) can be added. Different enzymes (for example, the enzyme that catalyzes a reaction that produces ATP from AMP and the enzyme that catalyzes a reaction that produces ATP from ADP) may share a common substrate. Depending on the type of enzyme, an additional component corresponding to the substrate need not be added. For example, when the enzyme that catalyzes a reaction that produces ADP from AMP is ADK, this enzymatic reaction is a reaction that produces ADP from ATP and AMP and therefore, does not require involving a further substrate for the enzymatic reaction in the liquid composition. The substrate is not particularly limited, and examples thereof include phosphoenolpyruvic acid and salts thereof, and pyrophosphoric acid and salts thereof for PPDK, and include phosphoenolpyruvic acid and salts thereof, acetylphosphoric acid and salts thereof, creatine phosphoric acid and salts thereof, polyphosphoric acid and salts thereof, and riboflavin phosphate and salts thereof for PK, AK, CK, PPK, and FMNK, respectively. Examples of the substrate for PFK1 and FBPase include fructose 1,6-bisphosphoric acid and salts thereof. Examples of the substrate for PWDK include phosphoenolpyruvic acid and salts thereof, and phosphoric acid and salts thereof, and examples of the substrate for ADP-dependent hexokinase include glucose. In one embodiment, the liquid composition described herein further comprises these substrates. In one embodiment, the concentration of a substrate (for example, phosphoenolpyruvic acid, pyrophosphoric acid, or a salt thereof) (or the respective concentrations or total concentration of substrates) in the liquid composition described herein can be, for example, 4 mM or lower, 3 mM or lower, 2.5 mM or lower, 2 mM or lower, 1.5 mM or lower, 1.2 mM or lower, 1 mM or lower, 0.5 mM or lower, 0.1 mM or lower, 0.05 mM or lower, 0.01 mM or lower, 0.001 mM or lower, or 0.0001 mM or lower. While a lower concentration of the substrate is capable of improving the stability of the liquid composition, the level of luminescence as a whole decreases. Therefore, if the level of luminescence is too low, the substrate(s) may be added before or during measurement. The concentration of the substrate(s) in the liquid composition during storage or the liquid composition supplemented with the substrate(s) before or during measurement can be, for example, 0.00001 mM or higher, 0.0001 mM or higher, 0.001 mM or higher, 0.01 mM or higher, 0.1 mM or higher or 1 mM or higher.

The concentration of the substrate (for example, phosphoenolpyruvic acid, pyrophosphoric acid, or a salt thereof) contained in the liquid composition can be measured on the basis of enzymatic reaction. For example, the concentrations of phosphoenolpyruvic acid (phosphoenolpyruvate) and pyrophosphoric acid (pyrophosphate) can be measured by producing pyruvic acid with AMP, phosphoenolpyruvic acid (phosphoenolpyruvate) and pyrophosphoric acid (pyrophosphate) as substrates using PPDK, and allowing lactate dehydrogenase and β-NADH to act thereon, followed by absorbance measurement at 340 nm. Alternatively, the concentrations of phosphoenolpyruvic acid (phosphoenolpyruvate) and pyrophosphoric acid (pyrophosphate) can also be measured by producing ATP with AMP, phosphoenolpyruvic acid (phosphoenolpyruvate) and pyrophosphoric acid (pyrophosphate) as substrates using PPDK, followed by luminescence measurement using luciferase. For example, the concentration of pyrophosphoric acid (pyrophosphate) can also be measured by producing ATP with pyrophosphoric acid (pyrophosphate) as a substrate using ATP sulfurylase, followed by luminescence level measurement using luciferase.

[Phosphoenolpyruvic acid (phosphoenolpyruvate) (PEP)]

In one embodiment, the liquid composition described herein comprises phosphoenolpyruvic acid (PEP) or a salt thereof. The measurement of ATP and AMP present in the system can be promoted by optionally adding an excess amount of PEP or a salt thereof to the system.

[Pyrophosphoric acid (pyrophosphate) (PPi)]

In one embodiment, the liquid composition described herein comprises pyrophosphoric acid (PPi) or a salt thereof. By optionally adding an excess amount of PPi or a salt thereof to the system, the measurement of ATP and AMP present in the system can be promoted.

[Cofactor]

In one embodiment, the liquid composition described herein comprises a cofactor. The cofactor refers to a non-protein chemical substance necessary for the catalytic activity of an enzyme. Examples of the cofactor include, but are not limited to, metal salts, vitamins and derivatives thereof, non-vitamin coenzymes, and organic prosthetic groups, for example, metal salts.

Examples of the cofactor of luciferase include metal salts, for example, salts of divalent metal ions such as magnesium and calcium, and manganese (for example, magnesium acetate). Examples of the cofactor of PPDK include metal salts, for example, magnesium. Those skilled in the art can determine the type and concentration of the cofactor (for example, a metal salt) according to the enzyme used.

In one embodiment, the concentration of the cofactor (for example, a metal (e.g., magnesium) salt) in the liquid composition described herein can be, for example, 30 mM or lower, 25 mM or lower, 20 mM or lower, 15 mM or lower, 10 mM or lower, 8 mM or lower, 6 mM or lower, 4 mM or lower, 2 mM or lower, 1 mM or lower, 0.5 mM or lower, 0.1 mM or lower, or 0.05 mM or lower. While a lower cofactor concentration is capable of improving the stability of the liquid composition, the level of luminescence as a whole decreases. Therefore, if the level of luminescence is too low, the cofactor may be added before or during measurement. The cofactor concentration of the liquid composition during storage or the liquid composition supplemented with the cofactor before or during measurement can be, for example, 0.0001 mM or higher, 0.001 mM or higher, 0.01 mM or higher, 0.1 mM or higher, 1 mM or higher or 5 mM or higher.

The concentration of the cofactor contained in the liquid composition can be measured by a general method known to those skilled in the art. The cofactor concentration can be measured, for example, by fractionating the liquid composition by chromatography such as HPLC, detecting a peak corresponding to the cofactor, and comparing the peak intensity with that of standard materials containing known concentrations of the cofactor. When the cofactor is a metal salt, its concentration can be measured by ICP emission spectrometry.

In one embodiment, the liquid composition described herein comprises an enzyme-stabilizing agent such as bovine serum albumin or gelatin that protects a reporter molecule such as luciferase from degradation. In one embodiment, the liquid composition described herein comprises a substance that adjusts pH or improves preservation. Examples of such substances include pH buffers (HEPES, Tricine, Tris, phosphate buffer solutions, acetate buffer solutions, and the like), reducing agents (dithiothreitol (DTT), 2-mercaptoethanol, and the like), and sugars (glucose, sucrose, trehalose, and the like).

[Addition of a component before or during measurement]

In one embodiment, the liquid composition does not contain at least one component necessary for cycling reaction, and the component that is not contained in the liquid composition is added to the liquid composition before or during measurement. In another embodiment, the liquid composition contains at least one component necessary for cycling reaction at a low concentration such that the cycling reaction rarely progresses or does not progress, and the component that is contained at a low concentration in the liquid composition is added to the liquid composition before or during measurement.

As used herein, the "cycling reaction" refers to a reaction system that measures ATP as well as ADP and/or AMP by directly or indirectly converting ADP and/or AMP to ATP.

As used herein, examples of the "component necessary for cycling reaction" include luciferase, luciferin, an enzyme that catalyzes a reaction that produces ATP from AMP and a substrate thereof, and a cofactor for the liquid compositions of the first and third aspects. composition of the first aspect. Examples of the "component necessary for cycling reaction" include luciferase, luciferin, an enzyme that catalyzes a reaction that produces ADP from AMP and a substrate thereof, an enzyme that catalyzes a reaction that produces ATP from ADP and a substrate thereof, and a cofactor for the liquid composition of the second aspect.

In one embodiment, the composition described herein does not contain at least one (for example, one, two, three, four, five, or all) of these components and contains other components, and the component(s) that is(are) not contained in the liquid composition is(are) added to the liquid composition before or during measurement.

As used herein, the term "before measurement" is not limited as long as the stability of the liquid composition is improved and before measurement can be, for example, 30 minutes before, 10 minutes before, 5 minutes before, 1 minute before, 30 seconds before, or 10 seconds before measurement of ATP and AMP and/or ADP, or immediately before the measurement. The term "during measurement" means the same time with measurement. A shorter time to measurement after addition of the component necessary for cycling reaction reduces the time for which cycling reaction occurs, and is thus capable of improving the stability of the liquid composition.

[Sample]

As used herein, the type of "sample" is not limited and can be, for example, a bio-related sample or a bio-related instrument, a blood-related sample or a blood-related instrument, or a cooking-related instrument.

As used herein, the bio-related sample encompasses any sample to which a biogenic substance may have been attached. As used herein, the bio-related instrument refers to any instrument to or in which a biogenic substance may be attached or remain. As used herein, examples of the environment from which a bio-related sample or a bio-related instrument is derived include the environment to or in which a biogenic liquid may be attached to and remain. Examples of the environment include, but are not limited to, clothing, protection tools such as gloves, a hand, a finger, a bed, a switch, a doorknob, a bed fence, a nurse call button, a handrail, a washroom, a washbowl, a rest room, and a toilet stool. The biogenic substance may be from a human or an animal. **In** one embodiment, the biogenic substance may be from a human. **In** one embodiment, the bio-related sample does not comprise a sample from a non-human animal and comprises a sample from a human. **In** one embodiment, the bio-related instrument does not comprise a non-human animal-related instrument and comprises a human body-related instrument.

Examples of the biogenic substance include liquids or solids. Examples of the liquids include, but are not limited to, body fluids, blood, lymph, sweat, nasal mucus, tear, saliva, digestive juice, tissue fluid, ascitic fluid, amniotic fluid, spinal fluid, urine, feces, vomiting, and sebum. Examples of the solids include, but are not limited to, materials that are originally solids, such as tissue pieces, pieces of meat, and cells as well as solidified liquids, coagulated blood, excrement, scurf, eye mucus, and scab.

Examples of the bio-related instrument include medical instruments, and examples thereof include surgical instruments; endoscopes (for example, upper endoscopes to be used for the examination of the esophagus, the stomach, and the duodenum; lower endoscopes to be used in the examination of the rectum and the large intestine; or double balloon small-bowel endoscopes, preferably lower endoscopes); catheters, scalpels, tubes to be inserted into the body of patients, instruments to be inserted into the body of patients, surgical instrument-washing tanks; and medical instrument-washing environments.

As used herein, the blood-related sample encompasses any sample to which blood may have been attached. As used herein, the blood-related instrument refers to any instrument to or in which blood may be attached or remain. Examples of the blood-related instrument include medical instruments to or in which blood may be attached or remain. Examples thereof include surgical instruments; endoscopes (for example, upper endoscopes to be used for the examination of the esophagus, the stomach, and the duodenum; lower endoscopes to be used in the examination of the rectum and the large intestine; or double balloon endoscopes, preferably lower endoscopes); catheters, scalpels, tubes to be inserted into the body of patients, instruments to be inserted into the body of patients, surgical instrument-washing tanks; and medical instrument-washing environments. As used herein, examples of the environment from which a blood-related sample or a blood-related instrument is derived include the environment to or in which blood may be attached or remain. Examples of the environment include an operating table, a washing tank, clothing, protection tools such as gloves, a hand, a finger, a bed, a handrail, a washroom, a washbowl, and medical facilities. Other examples include sites of accidents, sites of injury cases, and sites where bloodstain search is performed. The blood may be from a human or an animal. **In** one embodiment, the blood is from a human. **In** one embodiment, the blood does not comprise blood from animal meat or fish meat related to food.

Examples of the blood include whole blood, serum, plasma, bloods for blood transfusions, collected primary blood, and solutions obtained by diluting primary blood. The blood-related sample also encompasses a solution containing hemocytes (leukocytes, erythrocytes, platelets) or a sample to which the solution may have been attached.

**In** one embodiment, the blood-related sample does not encompass collected blood itself (referred to as the primary sample for convenience). **In** this embodiment, for example, the "solution containing hemocytes" encompassed in the blood-related sample does not encompass blood itself. **In** one embodiment, the blood-related sample refers to a secondary sample from an instrument or an environment that has been contacted with a primary sample. The secondary sample may be obtained by wiping an instrument or an environment that may have been contacted with a primary sample with a cotton swab or the like. **In** one embodiment, the method of the present invention examines whether blood is attached

or blood remains to or in a secondary sample. In one embodiment, the blood-related sample may be a sample in which blood present therein has been diluted by a washing treatment or the like.

As used herein, the cooking-related instrument refers to a cooking instrument and the environment of a cooking site or matter that is present in the environment and may be contaminated. Examples of cooking instruments include, but are not limited to, instruments for cooking and instruments related thereto, such as cutting boards, pots, frying pans, pressure cookers, iron boards, dishes, kitchen knives, cooking chopsticks, chopsticks, spoons, forks, knives, and other eating utensils, rice scoops, colander nets, sieve baskets, racks, cutting board holders, containers for storing cooking instruments, packaging containers, and packaging sheets. The environment of a cooking site refers to an environment such as a cooking site, a food processing plant, or a food providing facility, and examples of the matter that is present in the environment include, but are not limited to, equipment such as mixing tanks, piping, filling nozzles, and belt conveyors in food processing plants and the like, and sites or locations that are often touched by humans' hands, such as containers, door knobs, handles of instruments (refrigerators, ovens, and the like), switches, and phone receivers.

[Storage period]

The liquid composition described herein is directed to measuring ATP and AMP and/or ADP in a sample after storage of the liquid composition for 1 day or longer. The storage described herein includes the period from after the liquid composition is prepared to before the liquid composition is used in measurement as well as the storage period of a liquid composition produced during production of powder compositions. In this case, the liquid composition described herein is stored for a certain period after production, and converted into a powder composition by a drying step, and then reconstituted into a liquid composition, which can be used for measuring ATP and AMP and/or ADP in a sample (optionally, after being stored again). The storage period of the liquid composition (or the total period when the liquid composition is stored multiple times) is not limited and can be, for example, 1 day or longer, 2 days or longer, 3 days or longer, 7 days or longer, 14 days or longer, 30 days or longer, 60 days or longer, 90 days or longer, 120 days or longer, 150 days or longer, 180 days or longer, 210 days or longer, 240 days or longer, 270 days or longer, or 300 days or longer. Further, the storage period can be 600 days or shorter, 500 days or shorter, or 400 days or shorter. Since the liquid composition described herein can have improved stability, the stability improving effect can be more clearly exerted when the storage period is long.

[Kit]

The present disclosure further describes a kit for measuring ATP in a sample, comprising the liquid composition described herein. The kit may comprise at least one of an extracting solution (for example, water or a buffer solution, or water or a buffer solution containing a surfactant such as benzalkonium chloride), a buffer solution, an instrument necessary for a test, a control and an instruction for use, in addition to the liquid composition described herein.

This kit may comprise a sampling unit and a reaction unit. The sampling unit is not particularly limited as long as a sample can be collected. Examples thereof include cotton swabs, sponges, porous plastics, filter papers, nonwoven fabrics, and droppers. The sampling unit (sample obtaining unit) is preferably, for example, in a rod shape from the viewpoint of convenient sampling, and is particularly preferably in the shape of a rod having a fibrous or sponge like wiping part, for example, a cotton swab-like shape.

The reaction unit is the unit (site) where reaction occurs when the sample collected by the sampling unit contains ATP or degradation products thereof. In one instance, the reaction unit comprises the liquid composition described herein. The reaction unit is preferably a transparent container, which enables the level of luminescence to be measured directly.

The kit may comprise other units, for example, a storage unit or an extraction unit, in addition to the sampling unit and the reaction unit. When the liquid composition described herein does not contain at least one component (for example, luciferin) necessary for cycling reaction, the storage unit serves as a site where the component that is not contained in the liquid composition is stored. When the sample collected by the sampling unit contains ATP or degradation products thereof, the extraction unit serves as a site where ATP or degradation products thereof are extracted into the extract. The extraction unit may comprise an extract. The extraction unit can transfer the sample extracted by the extraction unit to the reaction unit where the sample is then reacted.

When the liquid composition described herein does not contain at least one component necessary for a cycling reaction, this kit may comprise the component that is not contained in the liquid composition. In this case, the liquid composition and the component that is not contained in the liquid composition can be stored separately. For example, the liquid composition may be contained in the reaction unit, and the component that is not contained in the liquid composition may be contained in the sampling unit, the storage unit, the extraction unit, or other units isolated from the liquid composition.

[Method]

In the third aspect, the present invention relates to a method for measuring ATP and AMP and/or ADP in a sample, comprising using the liquid composition described herein or use of the liquid composition described herein for measuring ATP and AMP and/or ADP in a sample.

ATP and AMP and/or ADP in the sample can be measured, for example, without limitation, by adding a sample solution containing ATP to the liquid composition described herein, and measuring luminescence. The amounts of the liquid composition and the sample solution may be the same or different. The amounts of the liquid composition and the sample solution are not limited and can each be, for example, 0.01 mL to 10 mL, 0.25 mL to 4 mL, 0.5 mL to 2 mL or 0.1 mL. The sample solution is not limited and can be obtained by suspending, in an extracting solution, a cotton swab or the like used to wipe the sample.

The level of luminescence can be measured using a known luminometer (Lumitester Smart, Lumitester PD-20, or Lumitester PD-30 manufactured by Kikkoman Biochemifa Company, or the like) or an apparatus with a photodiode (SystemSURE Plus or EnSURE manufactured by Hygiena, LLC, AccuPoint Advanced manufactured by Neogen Corporation, or the like), or an apparatus with a photomultiplier tube (Clean Trace LM1 or Clean Trace UNG3 manufactured by 3M Corporation, Lumitester C-110 or Lumitester C-100 manufactured by Kikkoman Biochemifa Company, Junior LB9509, CentroLB960 or Lumat3 LB9508 manufactured by Berthold Technologies GmbH & Co. KG, or the like). Luminescence can be expressed as the relative luminescence unit (RLU) compared to a defined standard. (For example, in the case of collecting the sample using a rod having a wiping part,) the value of the relative luminescence unit may be used directly in cleanliness management or the like. In this case, a standard solution having a known ATP concentration is not used. In one embodiment, the method described herein uses none of an ATP standard solution, an ADP standard solution, nor an AMP standard solution. When the relative luminescence unit obtained by measuring only the sample, and without using an ATP, ADP, and/or AMP (hereinafter, also referred to as ATP, etc.) standard solution, is used directly in cleanliness management or the like, use of the composition described herein, having excellent stability, is particularly highly advantageous because the decrease in luminescence level caused the instability of the liquid composition directly influences the examination results.

In another embodiment, the concentration of ATP, etc. in the sample solution may be measured by generating a calibration curve (standard curve) using a substrate solution whose concentration of ATP, etc. is known, then adding the liquid composition described herein to a sample solution whose concentration of ATP, etc. is unknown, and measuring luminescence in the same conditions. In this embodiment, even if the level of luminescence is decreased due instability of the liquid composition, by generating a calibration curve of ATP, etc. in each case using the liquid composition under the same conditions, the degree to which decrease in luminescence level becomes problematic can be relatively reduced.

Examples

The present invention is more specifically described referring to Examples below. However, the technical scope of the present invention is not limited at all by the Examples.

<Example 1: Luciferase concentration measurement>

(280 nm absorbance method)

The absorbance at 280 nm of a sample solution appropriately diluted with PBS was measured using absorbance spectrometer U-3900 (manufactured by Hitachi High-Tech Corporation) and this was taken as the luciferase concentration (mg protein/mL).

(Bradford method)

Protein concentration was measured using Coomassie (Bradford) Protein Assay Kit (manufactured by Thermo Fisher Scientific, Inc.) based on the Bradford method. Specifically, 100 μL of a protein measurement reagent (Quick Start Bradford 1 × Dye Reagent, manufactured by Thermo Fisher Scientific, Inc.) was added to 100 μL of the sample solution appropriately diluted with PBS, and absorbance at 595 nm was measured using MICROPLATE READER SH-9000 (manufactured by Corona Electric Co., Ltd.). A calibration curve was generated by measurement using 2 mg/mL Albumin Standard Ampules attached to the kit as a standard protein instead of the sample solution, and the amount of luciferase was determined.

(Conversion between 280 nm absorbance method and the Bradford method)

As a result of measurement, 1 mg protein/mL in the 280 nm absorbance method corresponded to 1.77 mg/mL in the Bradford method.

<Example 2: Preparation of a cycling luminescence reagent, and background luminescence level>

A solution having basic composition was newly designed on the basis of the alternative composition of LuciPac Pen (manufactured by Kikkoman Biochemifa Company (the same applies to description below); a kit that is used by adding an extraction reagent (solution) to a powder luminescence reagent) described in the paragraph [0094] of International Publication No. WO 2018/147443. The alternative composition of LuciPac Pen is as follows.

<Alternative composition of LuciPac Pen>

7 mM magnesium acetate, 0.5 mM luciferin, 25 mM tricine, 0.2 mg protein/mL luciferase (a value based on absorbance at 280 nm; hereinafter, described as the value 0.35 mg/mL based on the Bradford method according to Example 1), 0.2 mM potassium pyrophosphate, 1.4 mM potassium phosphoenolpyruvate, 1.3 U/mL PPDK

According to the manual of LuciPac Pen, a stainless surface was wiped with a cotton swab moistened with water, and then, the cotton swab was pushed through (the device) so that the powder luminescence reagent was dissolved in an extraction reagent (solution) that fell through. The amount of the solution here was 0.35 mL, and the pH was 7.8. On the basis of the obtained results and the aforementioned composition of LuciPac Pen, the solution having a basic composition was newly designed as follows.

<Basic composition>

7 mM magnesium acetate, 0.5 mM luciferin (manufactured by Biosynth AG; the same applies to description below), 25 mM tricine, 0.35 mg/mL (Bradford method) luciferase (HLK described in JP Patent Publication (Kokai) No. 11-239493 A (1999); the same applies to description below), 0.2 mM potassium pyrophosphate, 1.4 mM potassium phosphoenolpyruvate, 1.3 U/mL PPDK (PPDK described in JP Patent Publication (Kokai) No. 8-168375 A (1996), manufactured by Kikkoman Biochemifa Company; the same applies to description below), pH 7.8, the amount of the solution per luminescence measurement container: 0.35 mL

A luminescence reagent having the basic composition above was prepared, and 0.35 mL thereof was weighed into a luminescence reagent container (measurement tube, 10ϕ in the upper part × height of 35 mm; the same applies to description below) of LuciPac Pen, which was then installed onto the main body equipped with a cotton swab holder, followed by measurement using Lumitester Smart (manufactured by Kikkoman Biochemifa Company; the same applies to description below). The level of luminescence obtained here was taken as the background luminescence level. In order to convert the background luminescence level to an ATP concentration, 0.01 mL of a $1 \times 10^{-5}$ M ATP (manufactured by Oriental Yeast Co., Ltd.; the same applies to description below) solution was further added as a control, and the level of luminescence was measured after 10 seconds using Lumitester Smart. The level of luminescence obtained here was taken as the luminescence level at the time of ATP addition. The background luminescence level was subtracted from the luminescence level at the time of ATP addition to determine the Δlevel of luminescence.

Since the ATP concentration (M) of the solution supplemented with 0.01 mL of the $1 \times 10^{-5}$ M ATP solution (a total of 0.36 mL) was $2.77 \times 10^{-7}$, the background ATP + AMP concentration was determined according to the following calculation.

Background ATP + AMP concentration (M) = Background luminescence level (RLU) / ΔLevel of luminescence (RLU) $\times 2.77 \times 10^{-7}$ (M)

Table 1 shows the background luminescence level (RLU), the luminescence level at the time of ATP addition (RLU), the Δlevel of luminescence (RLU), and the background ATP + AMP concentration (M). RLU is an abbreviation of relative light units (relative luminescence level).

[Table 1]

| | |
|---|---|
| Background luminescence level (RLU) | 547 |
| Luminescence level at the time of ATP addition (RLU) | 559,489 |
| ΔLevel of luminescence (RLU) | 558,942 |

(continued)

| | |
|---|---|
| Background ATP + AMP concentration (M) | $2.7\times10^{-10}$ |

The background luminescence level is believed to be due to the contaminant ATP or AMP from the reagent, container, or instrument during preparation. In the case of preparation with usual care (due care) by those skilled in the art, the level of luminescence is in the order of 500 RLU, which corresponds to $2.7 \times 10^{-10}$ M (0.27 nM) in terms of the ATP + AMP concentration of the luminescence reagent, as set forth in Table 1. This concentration corresponds to a concentration obtained by adding 0.01 mL of an approximately $1 \times 10^{-8}$ M ATP solution to 0.35 mL of the reagent.
As for a reagent that is further supplemented with an enzyme that converts ADP into ATP or AMP and measures three components ATP + ADP + AMP, it is believed that the background luminescence level will be higher because ADP contaminant in the reagent is also measured and an enzyme that may be the cause of the contamination of ATP and ADP and AMP is added.

<Example 3: Change of luminescence level over time of a non-cycling luminescence reagent>

In order to prepare a non-cycling luminescence reagent, PPDK, which is an enzyme necessary for cycling, was excluded from the basic composition to prepare the reagent. 0.35 mL of the prepared solution was weighed into a luminescence reagent container of LuciPac Pen, to which 0.01 mL of a $1 \times 10^{-5}$ M ATP solution was then added. The container was installed onto the main body equipped with a cotton swab holder, followed by measurement using Lumitester Smart.
Figure 1 illustrates the change of luminescence level over time of the non-cycling luminescence reagent. As shown in Figure 1, in the non-cycling luminescence reagent, the added ATP was rapidly consumed by luminescent reaction through luciferin-luciferase reaction, and the level of luminescence was quickly attenuated. The light was almost quenched in approximately 2 minutes in the basic composition containing no PPDK.

<Example 4: Stability of cycling luminescence reagent containing each concentration of luciferin>

A cycling luminescence reagent containing each concentration of luciferin was prepared according to the basic composition except that the luciferin concentration was altered. Envisaging that the sample can be contaminated with ATP, 0.1 mL of a $1 \times 10^{-6}$ M ATP solution was added to 3.5 mL of the cycling luminescence reagent (having each luciferin concentration), and the mixture was left to stand at 25°C. At each time point, 0.36 mL of the mixture was weighed into a luminescence reagent container of LuciPac Pen, which was then installed onto the main body equipped with a cotton swab holder, followed by measurement using Lumitester Smart. The level of luminescence obtained here was taken as the background luminescence level. In order to further confirm the stability of the luminescence level, 0.01 mL of a $1 \times 10^{-5}$ M ATP solution was added thereto, and the level of luminescence was measured 10 seconds after addition using Lumitester Smart. The level of luminescence obtained here was taken as the luminescence level at the time of ATP addition, and the difference of the background luminescence level subtracted from this level of luminescence was taken as the Δlevel of luminescence.
Figure 2 illustrates the change of luminescence level over time of the cycling luminescence reagent containing each concentration of luciferin. Figure 3 illustrates the level of luminescence after 9 hours of the cycling reagent containing each concentration of luciferin in Figure 2.
A phenomenon in which the Δlevel of luminescence at the time of addition of ATP decreased over time was observed for the basic composition. In other words, this revealed that the cycling reagent had low stability. Further, a tendency was observed that such decrease in the Δlevel of luminescence at the time of addition of ATP was larger when the luciferin concentration was higher, and the decrease in luminescence level over time was suppressed by adjusting the luciferin concentration to a concentration lower than that in the basic composition. In general, with regard to measurement systems using enzymatic reactions, the substrate is added at a high concentration such that decrease in the level of the substrate with the progression of the enzymatic reaction does not influence the reaction rate. In particular, for cycling reactions, since the substrate is continuously being consumed, it is believed to be reasonable that a higher substrate concentration will lead to the stabilization of the reagent and the substrate should be added at a higher concentration. However, the results of this Example revealed that, surprisingly, in a cycling luminescence reagent that utilizes a luciferin-luciferase reaction, to the contrary, a low concentration of luciferin can stabilize the level of luminescence from the cycling luminescence reagent.

<Example 5: Stability of cycling luminescence reagent containing no luciferin>

Next, the stability improving effect was confirmed in a reagent that was stored in a luciferin-free state to which

luciferin was added immediately before reaction. A cycling luminescence reagent containing no luciferin was prepared according to the basic composition. Envisaging that the sample can be contaminated with ATP, 0.1 mL of a 1 × $10^{-6}$ M ATP solution was added to 3.5 mL thereof (hereinafter, the obtained mixture is referred to as a mixed solution).

(1) For the "Stored, including luciferin" case, 0.1 mL of a 17.5 mM luciferin solution (pH 7.8) (final concentration: 0.5 mM) was added to 3.6 mL of the mixed solution, which was further stored at 25°C. After storage for each duration, 0.37 mL of the mixed solution was weighed into a luminescence reagent container of LuciPac Pen, which was then installed onto the main body equipped with a cotton swab holder, followed by the measurement of the background luminescence level using Lumitester Smart. In order to further confirm the stability of luminescence level, 0.01 mL of a 1 × $10^{-5}$ M ATP solution was added as a control, and the level of luminescence was measured after 10 seconds using Lumitester Smart and taken as the luminescence level at the time of ATP addition. The difference of the background luminescence level subtracted from the luminescence level at the time of ATP addition was taken (regarded) as the Δlevel of luminescence.

(2) For the "Stored, without any luciferin" case, 3.6 mL of the mixed solution was stored at 25°C without the addition of a luciferin solution. After storage for each duration, 0.36 mL of the solution was weighed into a luminescence reagent container of LuciPac Pen, to which 0.01 mL of a 17.5 mM luciferin solution (pH 7.8) (final concentration: 0.5 mM) stored at 25°C was then added, followed by the measurement of the background luminescence level. Further, 0.01 mL of a 1 × $10^{-5}$ M ATP solution was added thereto, and the level of luminescence was measured after 10 seconds using Lumitester Smart and taken as the luminescence level at the time of ATP addition. The difference of the background luminescence level subtracted from the luminescence level at the time of ATP addition was taken as the Δlevel of luminescence.

Figure 4 illustrates the stability of the luminescence reagent containing luciferin or containing no luciferin. As shown in Figure 4, the level of luminescence from the reagent "stored, including luciferin" was decreased with the progression of cycling reaction during storage, whereas the level of luminescence from the reagent "stored, without any luciferin" was not decreased during storage and was kept constant. Thus, the stability was largely improved.

<Example 6: Stability of a non-cycling luminescence reagent containing no luciferin>

A reagent having the basic composition containing no PPDK, i.e., a non-cycling luminescence reagent, was (1) stored, including luciferin or (2) stored, without any luciferin in the same manner as in Example 5, and the stability of the luminescence level was confirmed and the residual rate of luminescence level after storage for 3 hours was shown. The residual rate of luminescence level after storage for 3 hours in the presence of cycling (Example 5) was also shown.

The results are shown in Figure 5. Decrease in luminescence level was not observed for any of the luminescence reagents that were stored, without any luciferin, and supplemented with luciferin immediately before measurement. By contrast, as for the reagents stored, including luciferin, decrease in luminescence level was observed only for the cycling luminescence reagent containing PPDK and was not observed for the reagent containing no PPDK in which cycling does not occur. In other words, the decrease in luminescence level observed in this Example is believed to be a phenomenon that does not occur in a luminescence reagent that utilizes a luciferin-luciferase reaction in which only ATP is to be measured and cycling does not occur, and is a phenomenon that occurs only when a luminescence reagent that utilizes a cycling reaction is stored in a liquid state.

<Example 7: The stability of the luminescence level of cycling luminescence reagents containing each concentration of luciferase>

From Examples 3 to 6, it was found that reduction in luminescence level is important for improving the stability of the luminescence level of a cycling luminescence reagent. Since it is also possible to reduce the level of luminescence by reducing the luciferase concentration, the relationship between the stability of the luminescence level and the luciferase concentration was examined in a cycling luminescence reagent.

A cycling luminescence reagent containing each concentration of luciferase was prepared according to the basic composition except that the luciferase concentration was altered. Envisaging that the sample can be contaminated with ATP, 0.1 mL of a 1 × $10^{-6}$ M ATP solution was added to 3.5 mL of the cycling luminescence reagent, and the mixture was left to stand at 25°C. At each time point, 0.36 mL of the mixture was weighed into a luminescence reagent container of LuciPac Pen, which was then installed onto the main body equipped with a cotton swab holder, followed by measurement using Lumitester Smart. The level of luminescence obtained here was taken as the background luminescence level. In order to further confirm the stability of luminescence level, 0.01 mL of a 1 × $10^{-5}$ M ATP solution was added as a control, and the level of luminescence was measured 10 seconds after addition using Lumitester Smart. The level of luminescence

obtained here was taken as the luminescence level at the time of ATP addition, and the difference of the background luminescence level subtracted from this level of luminescence was taken as the Δlevel of luminescence.

Figure 6 illustrates the residual luminescence level of the cycling luminescence reagent containing each concentration of luciferase. Figure 7 illustrates the level of luminescence after 9 hours of the cycling reagent having each luciferase concentration in Figure 6.

As shown in Figure 6, a tendency was observed such that the level of luminescence decreased over time with an increase in luciferase concentration, and the decrease in luminescence level over time was suppressed by adjusting the luciferase concentration to a concentration lower than that in the basic composition was found.

<Example 8: ATP concentration dependency of the decrease in the luminescence level of the cycling luminescence reagent>

In the presence of ATP in a measurement system, a cycling luminescence reagent is supposed to continue luminescence at a certain level of luminescence as long as the substrate luciferin is not depleted (no longer exists). Similar experimental results as in Examples 4 to 7 can be obtained by adding ATP before storage and observing change of luminescence level over time, even if ATP is not added after storage. Therefore, the stability of the luminescence reagent can be conveniently examined. In this Example, in order to construct an experimental system that does require adding ATP after storage, an appropriate ATP concentration is studied when an ATP solution was not added to the reagent after storage.

A cycling luminescence reagent was prepared according to the basic composition, and 0.35 mL thereof was weighed into a luminescence reagent container of LuciPac Pen, to which 0.01 mL of a $1 \times 10^{-5}$ to $1 \times 10^{-8}$ M ATP solution or sterile ultra pure water was then added, followed by luminescence level measurement over time using Lumitester Smart. The level of luminescence from the sample supplemented with sterile ultra pure water was subtracted from the level of luminescence from the sample supplemented with each concentration of ATP to determine the Δlevel of luminescence, which was illustrated in a graph form.

The results are shown in Figure 8. There was a correlation between the attenuation of luminescence level and the ATP concentration, and a tendency was observed such that the higher the ATP concentration the more attenuate the level of luminescence was and the lower the ATP concentration the less attenuate the level of luminescence was.

The amount of ATP in the cycling luminescence reagent prepared in Example 2 envisaging that the sample can be contaminated with ATP corresponds to 0.01 mL of approximately $1 \times 10^{-8}$ M ATP added. In the presence of ATP at this concentration level, the level of luminescence is not attenuated in 2 hours. Thus, the attenuation of luminescence level does not become a major problem when the cycling luminescence reagent is used up in a short period. However, when the regent is stored for a long period, this may increasingly become a problem. For example, provided that the attenuation of the luminescence level is proportional to the amount of luciferin consumed, i.e., the level of luminescence from luciferin, computationally, it is believed that, since the level of luminescence was attenuated to 30% in 2 hours by the addition of $1 \times 10^{-5}$ M ATP, the level of luminescence will be attenuated to this level in 20 hours under contamination with $1 \times 10^{-6}$ M ATP, in 200 hours (8.3 days) under contamination with $1 \times 10^{-7}$ M ATP, and in 2000 hours (83 days) under contamination with $1 \times 10^{-8}$ M ATP.

In the Examples described herein, in order to obtain results in a short period, a high concentration ATP solution was added. Although the stability of a measurement kit may be influenced by the stability of an enzyme, the stability of a substrate itself, or the like, evaluation at least for a short duration is considered to be possible without being influenced by the stability of the enzyme or substrate, or the like because a low concentration of ATP did not attenuate the level of luminescence.

<Example 9: The level of luminescence during storage from cycling luminescence reagents containing each concentration of luciferin, and the stability of the reagents>

As described in Example 8, the stability of a cycling luminescence reagent can be examined by adding ATP to the reagent and examining the change of luminescence level over time. Further, it was found that results can be obtained in a short time by adding a high concentration of ATP. Moreover, Examples 3 to 6, etc. revealed that it is important to suppress luminescent reaction during storage. In this Example, in order to suppress luminescent reaction during storage, an experiment was conducted to define a standard level of luminescence in stored state. First, the level of luminescence in stored state is defined, and then, experimental results on which the definition is based is described.

The level of luminescence (RLU) in stored state is defined as the level of luminescence (RLU) determined by adding a luminescence reagent in stored state to a 0.35 mL measurement tube, then adding 0.01 mL of a $1 \times 10^{-7}$ M ATP solution thereto, and leaving the mixture to stand at 25°C for 1 hour, followed by measurement using Lumitester Smart (manufactured by Kikkoman Biochemifa Company).

The experiment for determining the definition is as follows. A cycling luminescence reagent containing each

concentration of luciferin (components other than luciferin were the same as those in the basic composition) was prepared, and 0.35 mL thereof was weighed into a luminescence reagent container of LuciPac Pen, to which 0.01 mL of a $1 \times 10^{-5}$ to $1 \times 10^{-8}$ M ATP solution or sterile ultra pure water was further added, followed by luminescence level measurement over time using Lumitester Smart. The level of luminescence from the sample supplemented with sterile ultra pure water was subtracted from the level of luminescence from the sample supplemented with each concentration of ATP to determine the Δlevel of luminescence, which is shown in Figures 9 to 14.

As shown in Figures 9 and 10, decrease in luminescence level was observed when $1 \times 10^{-5}$ M ATP was added and results were obtained with a tendency similar to those in Example 4. It was confirmed that a test with a short duration conducted by this method enables long term storage stability to be predicted without being influenced by an enzyme or the degradation of the substrate itself.

When defining the level of luminescence during storage, for example, the effect of termination of cycling cannot be correctly evaluated merely by measuring the level of luminescence immediately after addition of ATP because, in the case of stopping the cycling, the level of luminescence is high immediately after the addition of ATP, as in Figure 1 of Example 3. By contrast, for example, at 1 hour from ATP addition, since ATP is degraded by luciferase, the level of luminescence during storage, including the effect of termination of cycling can be examined by examining the level of luminescence after 1 hour. However, as shown in Figures 9 to 12, decrease in luminescence level over time is observed with the addition of a $1 \times 10^{-5}$ M or $1 \times 10^{-6}$ M ATP solution and, therefore, the level of luminescence during storage cannot be accurately measured. By contrast, as shown in Figures 13 and 14, the level of luminescence is not attenuated for approximately 2 hours in the presence of $1 \times 10^{-7}$ M ATP and, therefore, the level of luminescence during storage can be stably examined. On the other hand, the Δlevel of luminescence in the presence of $1 \times 10^{-8}$ M ATP is equivalent to the background luminescence level described in Example 2 and is susceptible to this ATP and therefore, this concentration is considered inappropriate (data not shown).

On the basis of these results, the level of luminescence (RLU) in stored state was defined as described above. The level of luminescence (RLU) in stored state according to this definition is described in Table 2 below.

[Table 2]

| Luciferin concentration (mM) | Level of luminescence after 60 min (RLU) |
|---|---|
| 0.5 | 6032 |
| 0.33 | 5189 |
| 0.25 | 4543 |
| 0.17 | 3031 |
| 0.125 | 2790 |
| 0.1 | 2329 |
| 0.05 | 1268 |
| 0.02 | 571 |
| 0.01 | 308 |
| 0.005 | 183 |

From Figure 10, if the luciferin concentration is set to be lower than 0.5 mM (from Table 2, the level of luminescence in stored state is 6000 RLU) then a tendency is observed such that a decrease in the level of luminescence as compared with the basic composition is low and stability is improved. Further, from Figure 10, in particular, if the luciferin concentration is 0.1 mM or lower (from Table 2, the level of luminescence in stored state is 2300 RLU or less) it was found that stability could be improved much further.

<Example 10: Level of luminescence during storage from cycling luminescence reagent containing each concentration of luciferase, and stability of the reagent>

A cycling luminescence reagent containing each concentration of luciferase (components other than luciferase were the same as those in the basic composition) was prepared, and 0.35 mL thereof was weighed into a luminescence reagent container of LuciPac Pen, to which 0.01 mL of a $1 \times 10^{-5}$ to $1 \times 10^{-8}$ M ATP solution or sterile ultra pure water was further added, followed by luminescence level measurement over time using Lumitester Smart. The level of luminescence from the sample supplemented with sterile ultra pure water was subtracted from the level of luminescence from the sample supplemented with each concentration of ATP to determine the Δlevel of luminescence, which is shown in Figures 15 to 20.

Decrease in luminescence level was observed with the addition of the 1 × $10^{-5}$ M ATP solution and results were obtained with the same tendency as those in Example 7. It was confirmed that this method enables long term storage stability to be examined in a short period of time without being influenced by an enzyme or the degradation of a substrate itself. As shown in Figures 19 and 20, the level of luminescence is not attenuated for approximately 2 hours in the presence of the 1 × $10^{-7}$ M ATP solution and, therefore, the definition of the level of luminescence in stored state described in Example 9 is demonstrated to be reasonable according to this Example as well. The level of luminescence (U) in stored state according to this definition is described in Table 3 below.

[Table 3]

| Luciferase concentration (mg/mL) | Level of luminescence after 60 min (RLU) |
|---|---|
| 0.35 | 6440 |
| 0.07 | 1358 |
| 0.035 | 612 |

From Figure 15, if the luciferase concentration is set to be lower than 0.35 mg/mL (from Table 3, the level of luminescence in stored state is 6000 RLU or less), then a tendency is observed such that a decrease in the level of luminescence as compared with the basic composition is low and stability is improved. Further, from Table 3, at the concentration that brought about improvement (0.07 mg/mL or lower), the level of luminescence was found to fall below 2300 RLU 1 hour after addition of the 1 × $10^{-7}$ M ATP solution.

<Example 11: The stability of a luminescence reagent without cycling components>

The basic composition was partially altered, and a study was conducted by examining whether a stability improving effect can be obtained by excluding some of the substrates or enzymes related to cycling from the basic composition and adding the same during measurement.

A luminescence reagent was divided into solution A and solution B. One of the components necessary for luminescent reaction or cycling reaction (magnesium acetate, potassium phosphoenolpyruvate, potassium pyrophosphate, luciferin, luciferase, and PPDK) was transferred to solution B, and these solutions were prepared and stored separately. Immediately before reaction, 0.05 mL each of the solutions was mixed, and 0.1 mL of an ATP solution was added to the mixture, followed by luminescence level measurement.

Each of the solutions was prepared such that solution A of the luminescence reagent had a composition (pH 7.8) excluding any one of the components 24 mM Mg acetate, 1.6 mM luciferin, 4 mM potassium phosphoenolpyruvate (PEP), 0.4 mM potassium pyrophosphate (PPi), 1.0 mg/mL (Bradford method) luciferase, and 4.3 U/mL PPDK, and containing the remaining components in 50 mM tricine while solution B of the luminescence reagent had a composition (pH 7.8) containing 50 mM tricine supplemented with the any one component excluded from solution A, i.e., 24 mM magnesium acetate, 4 mM PEP, 0.4 mM PPi, 1.6 mM luciferin, 1.0 mg/mL (Bradford method) luciferase, or 4.3 U/mL PPDK.

The compositions of solution A and solution B are described in Tables 4 to 9 regarding a reagent excluding luciferase, a reagent excluding PPDK, a reagent excluding luciferin, a reagent excluding Mg acetate, a reagent excluding PEP, and a reagent excluding PPi, respectively.

[Table 4]

| Solution A | | Solution B | |
|---|---|---|---|
| 50 mM | Tricine | 50 mM | Tricine |
| 4.3 U/mL | PPDK | 1.0 mg/mL | Luciferase |
| 1.6 mM | Luciferin | | |
| 24 mM | Mg acetate | | |
| 4 mM | PEP | | |
| 0.4 mM | Pyrophosphate | | |

[Table 5]

| Solution A | | Solution B | |
|---|---|---|---|
| 50 mM | Tricine | 50 mM | Tricine |
| 1.0 mg/mL | Luciferase | 4.3 U/mL | PPDK |

(continued)

| Solution A | | Solution B |
|---|---|---|
| 1.6 mM | Luciferin | |
| 24 mM | Mg acetate | |
| 4 mM | PEP | |
| 0.4 mM | Pyrophosphate | |

[Table 6]

| Solution A | | Solution B | |
|---|---|---|---|
| 50 mM | Tricine | 50 mM | Tricine |
| 1.0 mg/mL | Luciferase | 1.6 mM | Luciferin |
| 4.3 U/mL | PPDK | | |
| 24 mM | Mg acetate | | |
| 4 mM | PEP | | |
| 0.4 mM | Pyrophosphate | | |

[Table 7]

| Solution A | | Solution B | |
|---|---|---|---|
| 50 mM | Tricine | 50 mM | Tricine |
| 1.0 mg/mL | Luciferase | 24 mM | Mg acetate |
| 4.3 U/mL | PPDK | | |
| 1.6 mM | Luciferin | | |
| 4 mM | PEP | | |
| 0.4 mM | Pyrophosphate | | |

[Table 8]

| Solution A | | Solution B | |
|---|---|---|---|
| 50 mM | Tricine | 50 mM | Tricine |
| 1.0 mg/mL | Luciferase | 4 mM | PEP |
| 4.3 U/mL | PPDK | | |
| 1.6 mM | Luciferin | | |
| 24 mM | Mg acetate | | |
| 0.4 mM | Pyrophosphate | | |

[Table 9]

| Solution A | | Solution B | |
|---|---|---|---|
| 50 mM | Tricine | 50 mM | Tricine |
| 1.0 mg/mL | Luciferase | 0.4 mM | Pyrophosphate |
| 4.3 U/mL | PPDK | | |
| 1.6 mM | Luciferin | | |
| 24 mM | Mg acetate | | |
| 4 mM | PEP | | |

For the "Mixed and then stored" case, 0.5 mL of solution B was added to 0.5 mL of solution A. and envisaging that the sample can be contaminated with ATP when prepared, 0.01 mL of a $1 \times 10^{-5}$ M ATP solution was added thereto, and the mixture was stored at 25°C for 40 hours. A 0.1 mL aliquot of the sample thus stored was collected, and 0.1 mL of $1 \times 10^{-6}$ M ATP was added thereto. The level of luminescence was measured 10 seconds after addition using Lumitester Smart.

For the "stored and then mixed (Mixed after storage)" case, envisaging that the sample can be contaminated with

ATP when prepared, 0.01 mL of a 1 × $10^{-5}$ M ATP solution was added to 0.5 mL of solution A, nothing was added to solution B, and these solutions were each stored at 25°C for 40 hours. After storage, 0.05 mL of solution B was added to 0.05 mL of solution A containing ATP, and 0.1 mL of 1 × $10^{-6}$ M ATP was added thereto. The level of luminescence was measured 10 seconds after addition using Lumitester Smart.

For comparison, as the level of luminescence before storage, the level of luminescence obtained by mixing 0.5 mL of solution A and 0.5 mL of solution B, then adding 0.01 mL of a 1 × $10^{-5}$ M ATP solution to the mixture, collecting a 0.1 mL aliquot of the sample without storage, and adding 0.1 mL of 1 × $10^{-6}$ M ATP thereto is also shown in Figure 21.

It was found that for each of luciferase, PPDK, luciferin, magnesium acetate (Mg acetate), PEP, and PPi, in the "mixed and then stored" case, the level of luminescence was decreased by the addition of ATP; and in the "stored and then mixed" case, a high level of luminescence was maintained.

In the case where luciferase involved in luminescent reaction or PPDK related to the cycling reaction was excluded, the cycling reaction did not progress and the stability was improved. Further, in the case where a cofactor or a substrate such as magnesium acetate, phosphoenolpyruvate, or pyrophosphate, or the like, was excluded, the stability was improved. The roles of magnesium acetate, phosphoenolpyruvate, and pyrophosphate in the cycling reaction are as follows.

The magnesium ion of magnesium acetate is a factor necessary for the reactions of luciferase and PPDK, and in a reagent containing no magnesium acetate, neither the luminescent reaction nor the cycling reaction progresses. Phosphoenolpyruvate is a substrate necessary for the reaction of PPDK, and pyrophosphate is a substrate necessary for the reaction of PPDK. In the case where phosphoenolpyruvate or pyrophosphate is excluded, luminescent reaction due to contaminant ATP progresses. However, the light is quenched when the ATP is consumed, and the luminescent reaction no longer progresses thereafter.

These results demonstrated that terminating the reaction by separately storing a component necessary for the luciferase reaction or cycling reaction is also effective for improving the stability.

## Claims

1. Use of a liquid composition in measuring ATP and AMP and/or ADP in a sample after storage of the liquid composition, wherein the liquid composition has been stored 1 day or longer, and wherein

    (i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
    (ii) at least one or more of the following conditions are satisfied:

        the concentration of luciferin in the liquid composition is 0.4 mM or lower;
        the concentration of luciferase in the liquid composition based on the Bradford method is 0.3 mg/mL or lower;
        the concentration of the enzyme that catalyzes a reaction that produces ATP from AMP in the liquid composition is 1 U/mL or lower;
        the concentration of the substrate of the enzyme that catalyzes a reaction that produces ATP from AMP in the liquid composition is 0.1 mM or lower; and
        the concentration of the cofactor in the liquid composition is 6 mM or lower.

2. The use according to claim 1, wherein the liquid composition further comprises at least one component selected from an enzyme that catalyzes a reaction that produces ATP from ADP, a substrate of the enzyme that catalyzes a reaction that produces ATP from ADP, an enzyme that catalyzes a reaction that produces AMP from ADP, and a substrate of the enzyme that catalyzes a reaction that produces AMP from ADP, or at least one of these components is added to the liquid composition before or during measurement.

3. Use of a liquid composition in measuring ATP and AMP and/or ADP in a sample after storage of the liquid composition, wherein the liquid composition has been stored 1 day or longer, and wherein

    (i) the liquid composition comprises luciferase, luciferin, an enzyme that catalyzes a reaction that produces ADP from AMP, a substrate of the enzyme that catalyzes a reaction that produces ADP from AMP, an enzyme that catalyzes a reaction that produces ATP from ADP, a substrate of the enzyme that catalyzes a reaction that produces ATP from ADP, and a cofactor, or when at least one of these components is not contained in the liquid composition, the component that is not contained in the liquid composition is added thereto before or during

measurement, and
(ii) at least one or more of the following conditions are satisfied:

the concentration of the enzyme that catalyzes a reaction that produces ADP from AMP in the liquid composition is 450 U/mL or lower;
the concentration of the substrate of the enzyme that catalyzes a reaction that produces ADP from AMP in the liquid composition is 0.1 mM or lower;
the concentration of the enzyme that catalyzes a reaction that produces ATP from ADP in the liquid composition is 20 U/mL or lower;
the concentration of the substrate of the enzyme that catalyzes a reaction that produces ATP from ADP is 1.2 mM or lower; and
the concentration of the cofactor in the liquid composition is 6 mM or lower.

**4.** The use according to any one of claims 1 to 3, wherein the liquid composition has been stored 30 days or longer.

**5.** The use according to any one of claims 1 to 4, wherein the liquid composition does not contain at least one component selected from luciferase, luciferin, an enzyme that catalyzes a reaction that produces ATP from AMP, a substrate of the enzyme that catalyzes a reaction that produces ATP from AMP, an enzyme that catalyzes a reaction that produces ADP from AMP, a substrate of the enzyme that catalyzes a reaction that produces ADP from AMP, an enzyme that catalyzes a reaction that produces ATP from ADP, a substrate of the enzyme that catalyzes a reaction that produces ATP from ADP, and a cofactor, and the component that is not contained in the liquid composition is added thereto before or during measurement.

**6.** The use according to any one of claims 1 to 5, wherein the concentration of luciferin in the liquid composition is 0.4 mM or lower, and/or the concentration of luciferase based on the Bradford method therein is 0.3 mg/mL or lower.

**7.** The use according to any one of claims 1 to 6, wherein the concentration of luciferin in the liquid composition is 0.1 mM or lower.

**8.** The use according to any one of claims 1 to 7, wherein the concentration of luciferase in the liquid composition based on the Bradford method is 0.1 mg/mL or lower.

**9.** A method for measuring ATP and AMP and/or ADP in a sample, comprising bringing a liquid composition into contact with a sample and measuring the level of luminescence, wherein the liquid composition is for measuring ATP and AMP and/or ADP in a sample after storage of the liquid composition, wherein the liquid composition has been stored 1 day or longer, and wherein

(i) the liquid composition comprises luciferase, luciferin, an enzyme that catalyzes a reaction that produces ATP from AMP, a substrate of the enzyme that catalyzes a reaction that produces ATP from AMP, and a cofactor, or when at least one of these components is not contained in the liquid composition, the component that is not contained in the liquid composition is added to the liquid composition before or during measurement, and
(ii) at least one or more of the following conditions are satisfied:

the concentration of luciferin in the liquid composition is 0.4 mM or lower;
the concentration of luciferase in the liquid composition based on the Bradford method is 0.3 mg/mL or lower;
the concentration of the enzyme that catalyzes a reaction that produces ATP from AMP in the liquid composition is 1 U/mL or lower;
the concentration of the substrate of the enzyme that catalyzes a reaction that produces ATP from AMP in the liquid composition is 0.1 mM or lower; and
the concentration of the cofactor in the liquid composition is 6 mM or lower.

**10.** The method according to claim 9, wherein an ATP standard solution is not used.

## Patentansprüche

**1.** Verwendung einer flüssigen Zusammensetzung zur Messung von ATP und AMP und/oder ADP in einer Probe nach Lagerung der flüssigen Zusammensetzung, wobei die flüssige Zusammensetzung 1 Tag oder länger gelagert wurde,

und wobei

(i) die flüssige Zusammensetzung Luciferase, Luciferin, ein Enzym, das eine Reaktion katalysiert, bei der aus AMP ATP erzeugt wird, ein Substrat des Enzyms, das eine Reaktion katalysiert, bei der aus AMP ATP erzeugt wird, sowie einen Cofaktor umfasst, oder, falls mindestens eine dieser Komponenten nicht in der flüssigen Zusammensetzung enthalten ist, die Komponente, die nicht in der flüssigen Zusammensetzung enthalten ist, der flüssigen Zusammensetzung vor oder während der Messung zugesetzt wird, und
(ii) mindestens eine oder mehrere der folgenden Bedingungen erfüllt sind:

die Konzentration an Luciferin in der flüssigen Zusammensetzung beträgt 0,4 mM oder weniger;
die Konzentration an Luciferase in der flüssigen Zusammensetzung, die auf der Bradford-Methode basiert, beträgt 0,3 mg/ml oder weniger;
die Konzentration des Enzyms, das eine Reaktion katalysiert, bei der aus AMP ATP erzeugt wird, in der flüssigen Zusammensetzung beträgt 1 U/ml oder weniger;
die Konzentration des Substrats des Enzyms, das eine Reaktion katalysiert, bei der aus AMP ATP erzeugt wird, in der flüssigen Zusammensetzung beträgt 0,1 mM oder weniger; und
die Konzentration des Cofaktors in der flüssigen Zusammensetzung beträgt 6 mM oder weniger.

**2.** Verwendung nach Anspruch 1, wobei die flüssige Zusammensetzung ferner mindestens eine Komponente umfasst, die ausgewählt ist aus einem Enzym, das eine Reaktion katalysiert, bei der aus ADP ATP erzeugt wird, einem Substrat des Enzyms, das eine Reaktion katalysiert, bei der aus ADP ATP erzeugt wird, einem Enzym, das eine Reaktion katalysiert, bei der aus ADP AMP erzeugt wird, und einem Substrat des Enzyms, das eine Reaktion katalysiert, bei der aus ADP AMP erzeugt wird, oder wobei mindestens eine dieser Komponenten der flüssigen Zusammensetzung vor oder während der Messung zugesetzt wird.

**3.** Verwendung einer flüssigen Zusammensetzung zur Messung von ATP und AMP und/oder ADP in einer Probe nach Lagerung der flüssigen Zusammensetzung, wobei die flüssige Zusammensetzung 1 Tag oder länger gelagert wurde, und wobei

(i) die flüssige Zusammensetzung Luciferase, Luciferin, ein Enzym, das eine Reaktion katalysiert, bei der aus AMP ADP erzeugt wird, ein Substrat des Enzyms, das eine Reaktion katalysiert, bei der aus AMP ADP erzeugt wird, ein Enzym, das eine Reaktion katalysiert, bei der aus ADP ATP erzeugt wird, ein Substrat des Enzyms, das eine Reaktion katalysiert, bei der aus ADP ATP erzeugt wird, sowie einen Cofaktor umfasst, oder, falls mindestens eine dieser Komponenten nicht in der flüssigen Zusammensetzung enthalten ist, die Komponente, die nicht in der flüssigen Zusammensetzung enthalten ist, vor oder während der Messung zu dieser hinzugefügt wird, und
(ii) mindestens eine oder mehrere der folgenden Bedingungen erfüllt sind:

die Konzentration des Enzyms, das eine Reaktion katalysiert, bei der aus AMP ADP erzeugt wird, in der flüssigen Zusammensetzung beträgt 450 U/ml oder weniger;
die Konzentration des Substrats des Enzyms, das eine Reaktion katalysiert, bei der aus AMP ADP erzeugt wird, in der flüssigen Zusammensetzung beträgt 0,1 mM oder weniger;
die Konzentration des Enzyms, das eine Reaktion katalysiert, bei der aus ADP ATP erzeugt wird, in der flüssigen Zusammensetzung beträgt 20 U/ml oder weniger;
die Konzentration des Substrats des Enzyms, das eine Reaktion katalysiert, bei der aus ADP ATP erzeugt wird, beträgt 1,2 mM oder weniger; und
die Konzentration des Cofaktors in der flüssigen Zusammensetzung beträgt 6 mM oder weniger.

**4.** Verwendung nach einem der Ansprüche 1 bis 3, wobei die flüssige Zusammensetzung 30 Tage oder länger gelagert wurde.

**5.** Verwendung nach einem der Ansprüche 1 bis 4, wobei die flüssige Zusammensetzung nicht mindestens eine Komponente enthält, die ausgewählt ist aus Luciferase, Luciferin, einem Enzym, das eine Reaktion katalysiert, bei der aus AMP ATP erzeugt wird, einem Substrat des Enzyms, das eine Reaktion katalysiert, bei der aus AMP ATP erzeugt wird, einem Enzym, das eine Reaktion katalysiert, bei der aus AMP ADP erzeugt wird, einem Substrat des Enzyms, das eine Reaktion katalysiert, bei der aus AMP ADP erzeugt wird, einem Enzym, das eine Reaktion katalysiert, bei der aus ADP ATP erzeugt wird, einem Substrat des Enzyms, das eine Reaktion katalysiert, bei der aus ADP ATP erzeugt wird, sowie einem Cofaktor, und wobei die Komponente, die nicht in der flüssigen Zusammensetzung enthalten ist, vor oder während der Messung zu dieser hinzugefügt wird.

6. Verwendung nach einem der Ansprüche 1 bis 5, wobei die Konzentration an Luciferin in der flüssigen Zusammensetzung 0,4 mM oder weniger beträgt und/oder die Konzentration an Luciferase, die auf der Bradford-Methode basiert, in der flüssigen Zusammensetzung 0,3 mg/ml oder weniger beträgt.

7. Verwendung nach einem der Ansprüche 1 bis 6, wobei die Konzentration an Luciferin in der flüssigen Zusammensetzung 0,1 mM oder weniger beträgt.

8. Verwendung nach einem der Ansprüche 1 bis 7, wobei die Konzentration an Luciferase in der flüssigen Zusammensetzung, die auf der Bradford-Methode basiert, 0,1 mg/ml oder weniger beträgt.

9. Verfahren zur Messung von ATP und AMP und/oder ADP in einer Probe, umfassend das Inkontaktbringen einer flüssigen Zusammensetzung mit einer Probe und das Messen des Lumineszenzniveaus, wobei die flüssige Zusammensetzung zur Messung von ATP und AMP und/oder ADP in einer Probe nach Lagerung der flüssigen Zusammensetzung dient, wobei die flüssige Zusammensetzung 1 Tag oder länger gelagert wurde, und wobei

(i) die flüssige Zusammensetzung Luciferase, Luciferin, ein Enzym, das eine Reaktion katalysiert, bei der aus AMP ATP erzeugt wird, ein Substrat des Enzyms, das eine Reaktion katalysiert, bei der aus AMP ATP erzeugt wird, sowie einen Cofaktor umfasst, oder, falls mindestens eine dieser Komponenten nicht in der flüssigen Zusammensetzung enthalten ist, die Komponente, die nicht in der flüssigen Zusammensetzung enthalten ist, der flüssigen Zusammensetzung vor oder während der Messung zugesetzt wird, und
(ii) mindestens eine oder mehrere der folgenden Bedingungen erfüllt sind:

die Konzentration an Luciferin in der flüssigen Zusammensetzung beträgt 0,4 mM oder weniger;
die Konzentration an Luciferase in der flüssigen Zusammensetzung, die auf der Bradford-Methode basiert, beträgt 0,3 mg/ml oder weniger;
die Konzentration des Enzyms, das eine Reaktion katalysiert, bei der aus AMP ATP erzeugt wird, in der flüssigen Zusammensetzung beträgt 1 U/ml oder weniger;
die Konzentration des Substrats des Enzyms, das eine Reaktion katalysiert, bei der aus AMP ATP erzeugt wird, in der flüssigen Zusammensetzung beträgt 0,1 mM oder weniger; und
die Konzentration des Cofaktors in der flüssigen Zusammensetzung beträgt 6 mM oder weniger.

10. Verfahren nach Anspruch 9, wobei keine ATP-Standardlösung verwendet wird.

## Revendications

1. Utilisation d'une composition liquide pour mesurer l'ATP et l'AMP et/ou l'ADP dans un échantillon après stockage de la composition liquide, ladite composition liquide ayant été stockée pendant 1 jour ou plus, et :

(i) ladite composition liquide comprenant de la luciférase, de la luciférine, une enzyme qui catalyse une réaction produisant de l'ATP à partir d'AMP, un substrat de l'enzyme qui catalyse une réaction produisant de l'ATP à partir d'AMP, et un cofacteur, ou lorsqu'au moins un de ces composants n'est pas contenu dans la composition liquide, ce composant qui n'est pas contenu dans la composition liquide étant ajouté à la composition liquide avant ou pendant la mesure ; et
(ii) au moins une ou plusieurs des conditions suivantes étant satisfaites :

la concentration en luciférine dans la composition liquide est de 0,4 mM ou moins ;
la concentration en luciférase dans la composition liquide, d'après la méthode Bradford, est de 0,3 mg/mL ou moins ;
la concentration en enzyme qui catalyse une réaction produisant de l'ATP à partir d'AMP dans la composition liquide est de 1 U/mL ou moins ;
la concentration en substrat de l'enzyme qui catalyse une réaction produisant de l'ATP à partir d'AMP dans la composition liquide est de 0,1 mM ou moins ; et
la concentration en cofacteur dans la composition liquide est de 6 mM ou moins.

2. Utilisation selon la revendication 1, ladite composition liquide comprenant en outre au moins un composant choisi parmi une enzyme qui catalyse une réaction produisant de l'ATP à partir d'ADP, un substrat de l'enzyme qui catalyse une réaction produisant de l'ATP à partir d'ADP, une enzyme qui catalyse une réaction produisant de l'AMP à partir

d'ADP, et un substrat de l'enzyme qui catalyse une réaction produisant de l'AMP à partir d'ADP, ou au moins un de ces composants étant ajouté à la composition liquide avant ou pendant la mesure.

3. Utilisation d'une composition liquide pour mesurer l'ATP et l'AMP et/ou l'ADP dans un échantillon après stockage de la composition liquide, ladite composition liquide ayant été stockée pendant 1 jour ou plus, et :

(i) ladite composition liquide comprenant de la luciférase, de la luciférine, une enzyme qui catalyse une réaction produisant de l'ADP à partir d'AMP, un substrat de l'enzyme qui catalyse une réaction produisant de l'ADP à partir d'AMP, une enzyme qui catalyse une réaction produisant de l'ATP à partir d'ADP, un substrat de l'enzyme qui catalyse une réaction produisant de l'ATP à partir d'ADP, et un cofacteur, ou lorsqu'au moins un de ces composants n'est pas contenu dans la composition liquide, le composant qui n'est pas contenu dans la composition liquide étant ajouté à celle-ci avant ou pendant la mesure, et
(ii) au moins une ou plusieurs des conditions suivantes étant satisfaites :

la concentration en enzyme qui catalyse une réaction produisant de l'ADP à partir d'AMP dans la composition liquide est de 450 U/mL ou moins ;
la concentration en substrat de l'enzyme qui catalyse une réaction produisant de l'ADP à partir d'AMP dans la composition liquide est de 0,1 mM ou moins ;
la concentration en enzyme qui catalyse une réaction produisant de l'ATP à partir d'ADP dans la composition liquide est de 20 U/mL ou moins ;
la concentration en substrat de l'enzyme qui catalyse une réaction produisant de l'ATP à partir d'ADP est de 1,2 mM ou moins ; et
la concentration en cofacteur dans la composition liquide est de 6 mM ou moins.

4. Utilisation selon l'une quelconque des revendications 1 à 3, ladite composition liquide ayant été stockée pendant 30 jours ou plus.

5. Utilisation selon l'une quelconque des revendications 1 à 4, ladite composition liquide ne contenant pas au moins un composant choisi parmi la luciférase, la luciférine, une enzyme qui catalyse une réaction produisant de l'ATP à partir d'AMP, un substrat de l'enzyme qui catalyse une réaction produisant de l'ATP à partir d'AMP, une enzyme qui catalyse une réaction produisant de l'ADP à partir d'AMP, un substrat de l'enzyme qui catalyse une réaction produisant de l'ADP à partir d'AMP, une enzyme qui catalyse une réaction produisant de l'ATP à partir d'ADP, un substrat de l'enzyme qui catalyse une réaction produisant de l'ATP à partir d'ADP, et un cofacteur, et le composant qui n'est pas contenu dans la composition liquide étant ajouté à celle-ci avant ou pendant la mesure.

6. Utilisation selon l'une quelconque des revendications 1 à 5, ladite concentration en luciférine dans la composition liquide étant de 0,4 mM ou moins, et/ou la concentration en luciférase dans celle-ci, d'après la méthode Bradford, étant de 0,3 mg/mL ou moins.

7. Utilisation selon l'une quelconque des revendications 1 à 6, ladite concentration en luciférine dans la composition liquide étant de 0,1 mM ou moins.

8. Utilisation selon l'une quelconque des revendications 1 à 7, ladite concentration en luciférase dans la composition liquide, d'après la méthode Bradford, étant de 0,1 mg/mL ou moins.

9. Procédé de mesure de l'ATP et de l'AMP et/ou de l'ADP dans un échantillon, comprenant la mise en contact de la composition liquide avec un échantillon et la mesure du niveau de luminescence, la composition liquide étant destinée à la mesure de l'ATP et de l'AMP et/ou de l'ADP dans un échantillon après stockage de la composition liquide, ladite composition liquide ayant été stockée pendant 1 jour ou plus, et dans lequel :

(i) la composition liquide comprend de la luciférase, de la luciférine, une enzyme qui catalyse une réaction produisant de l'ATP à partir d'AMP, un substrat de l'enzyme qui catalyse une réaction produisant de l'ATP à partir d'AMP, et un cofacteur, ou lorsqu'au moins un de ces composants n'est pas contenu dans la composition liquide, le composant qui n'est pas contenu dans la composition liquide est ajouté à la composition liquide avant ou pendant la mesure, et
(ii) au moins une ou plusieurs des conditions suivantes sont satisfaites :

la concentration en luciférine dans la composition liquide est de 0,4 mM ou moins ;

la concentration en luciférase dans la composition liquide, d'après la méthode Bradford, est de 0,3 mg/mL ou moins ;
la concentration en enzyme qui catalyse une réaction produisant de l'ATP à partir d'AMP dans la composition liquide est de 1 U/mL ou moins ;
la concentration en substrat de l'enzyme qui catalyse une réaction produisant de l'ATP à partir d'AMP dans la composition liquide est de 0,1 mM ou moins ; et
la concentration en cofacteur dans la composition liquide est de 6 mM ou moins.

**10.** Procédé selon la revendication 9, dans lequel aucune solution étalon d'ATP n'est utilisée.

Fig. 1

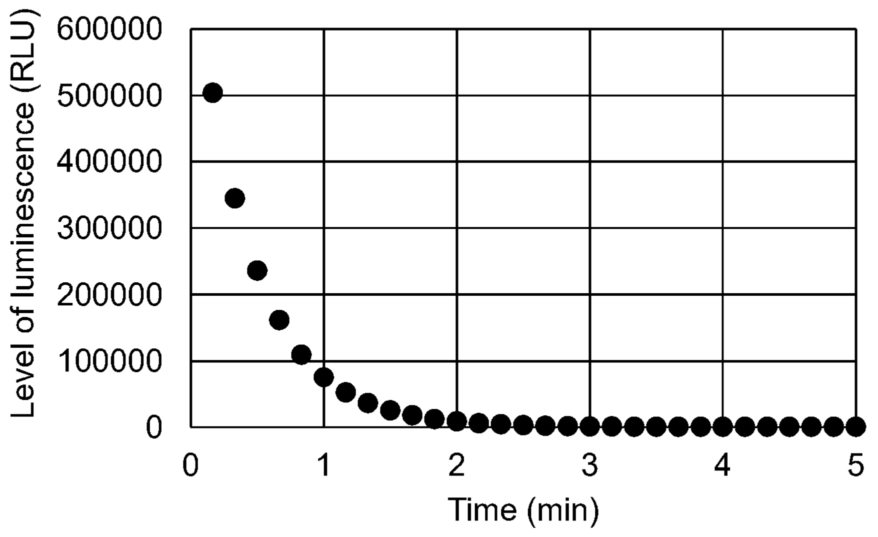
Level of luminescence (RLU)
600000
500000
400000
300000
200000
100000
0
0
1
2
3
4
5
Time (min)

Fig. 2

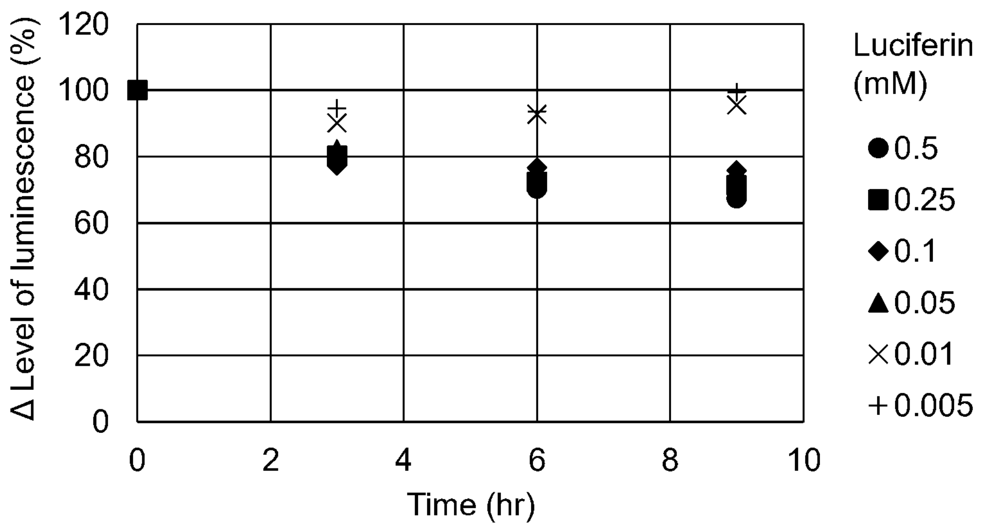
Δ Level of luminescence (%)
120
100
80
60
40
20
0
0
2
4
6
8
10
Time (hr)
Luciferin (mM)
0.5
0.25
0.1
0.05
0.01
0.005

Fig. 3

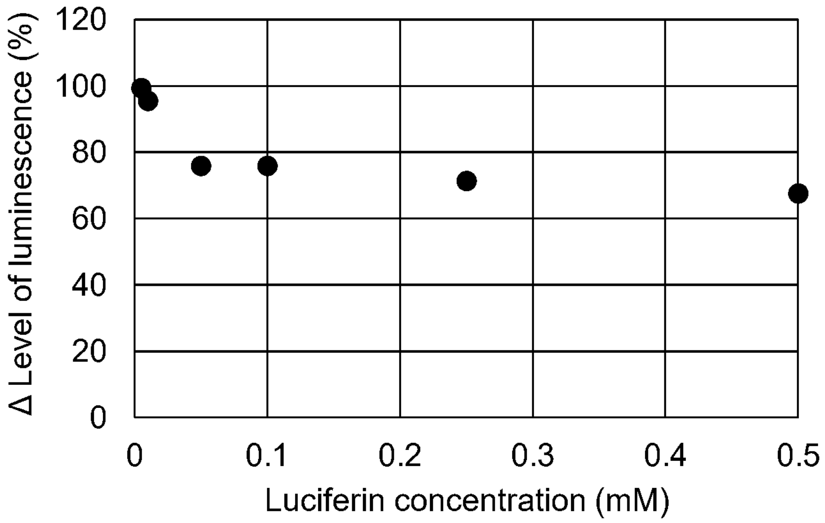
Δ Level of luminescence (%)
120
100
80
60
40
20
0
0
0.1
0.2
0.3
0.4
0.5
Luciferin concentration (mM)

Fig. 4

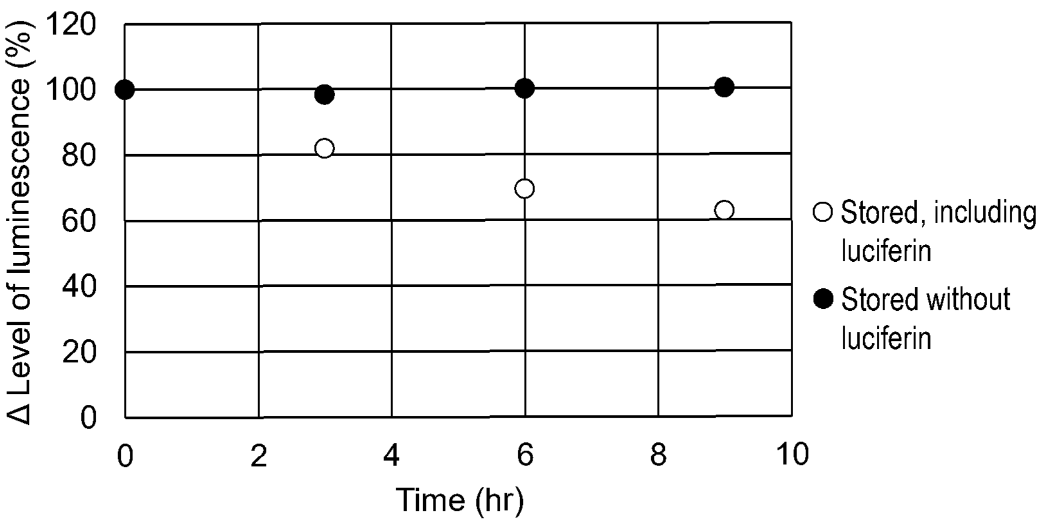

Δ Level of luminescence (%)
120
100
80
60
40
20
0
0
2
4
6
8
10
Time (hr)
○ Stored, including luciferin
● Stored without luciferin

Fig. 5

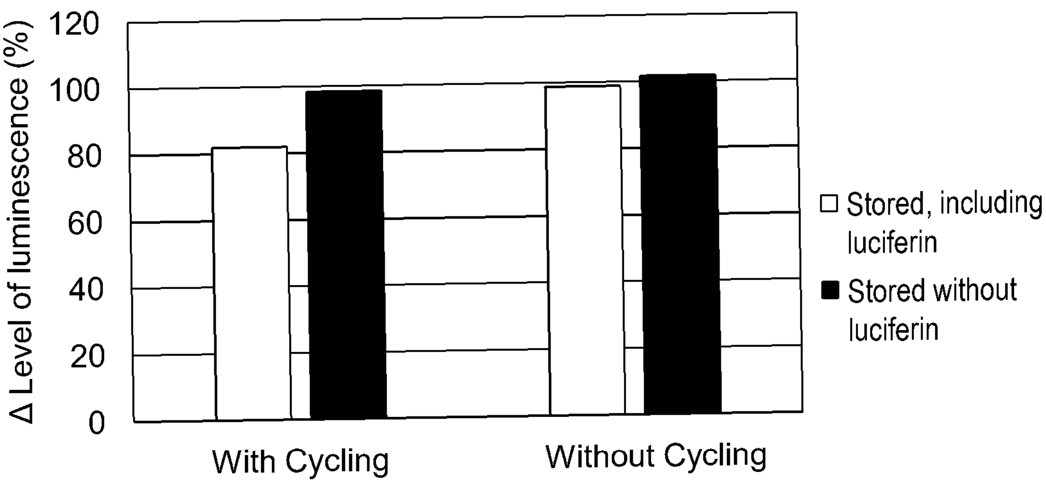
Δ Level of luminescence (%)
120
100
80
60
40
20
0
With Cycling
Without Cycling
Stored, including luciferin
Stored without luciferin

Fig. 6

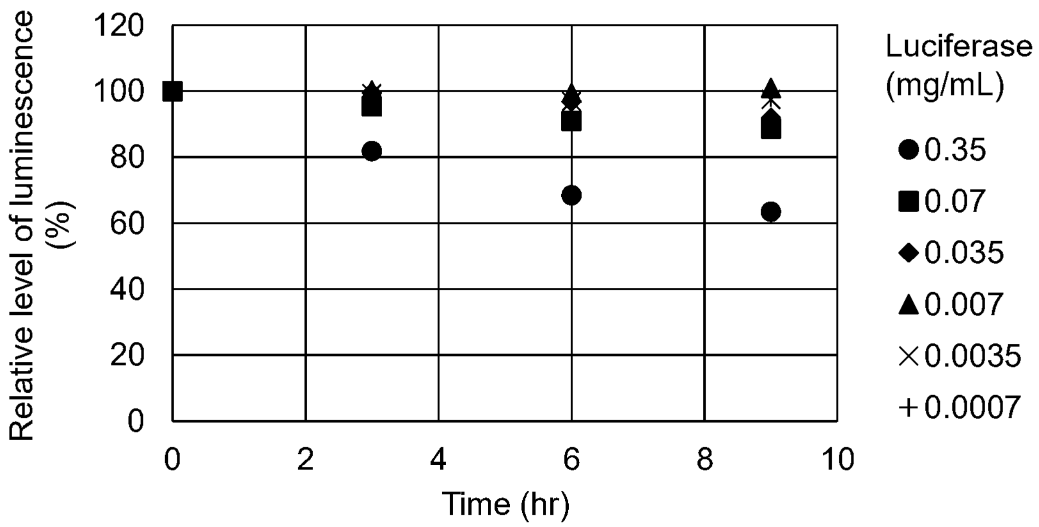
Relative level of luminescence (%)
120
100
80
60
40
20
0
0
2
4
6
8
10
Time (hr)
Luciferase (mg/mL)
0.35
0.07
0.035
0.007
0.0035
0.0007

Fig. 7

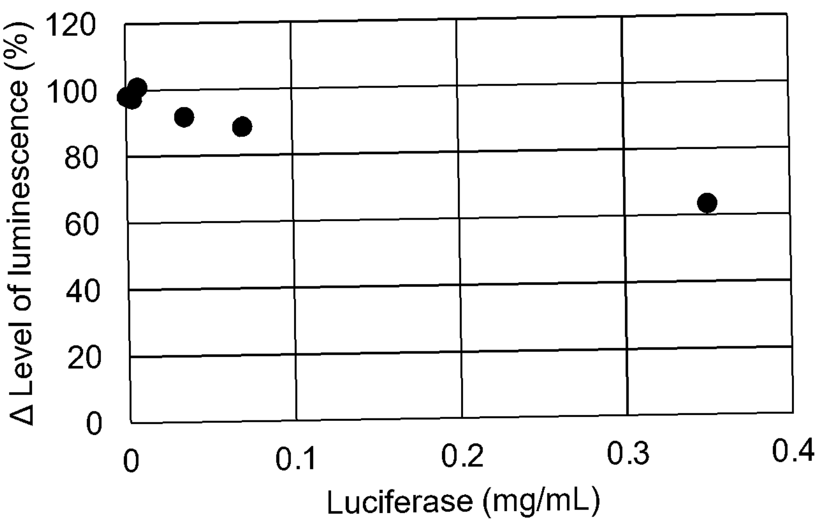
Δ Level of luminescence (%)
120
100
80
60
40
20
0
0
0.1
0.2
0.3
0.4
Luciferase (mg/mL)

Fig. 8

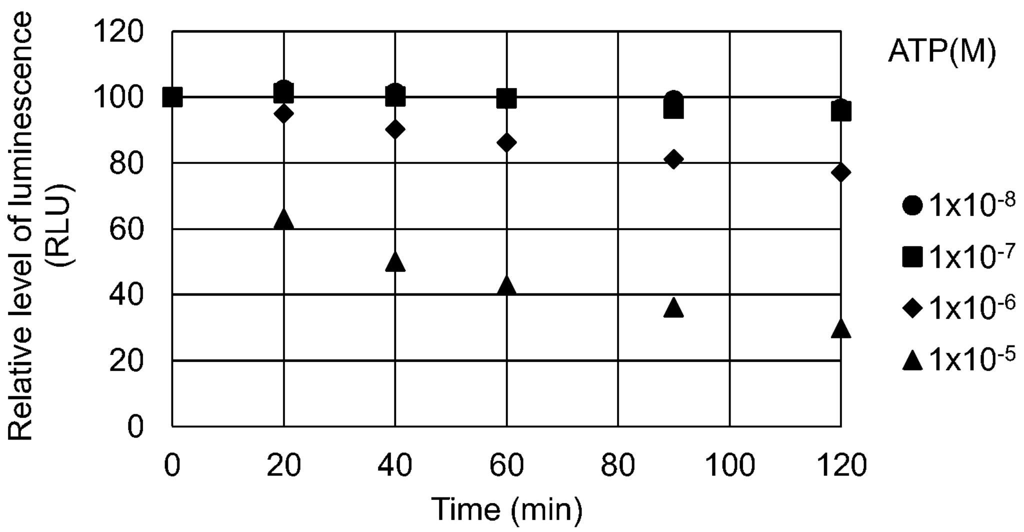
Relative level of luminescence (RLU)
120
100
80
60
40
20
0
0
20
40
60
80
100
120
Time (min)
ATP(M)
1x10-8
1x10-7
1x10-6
1x10-5

Fig. 9

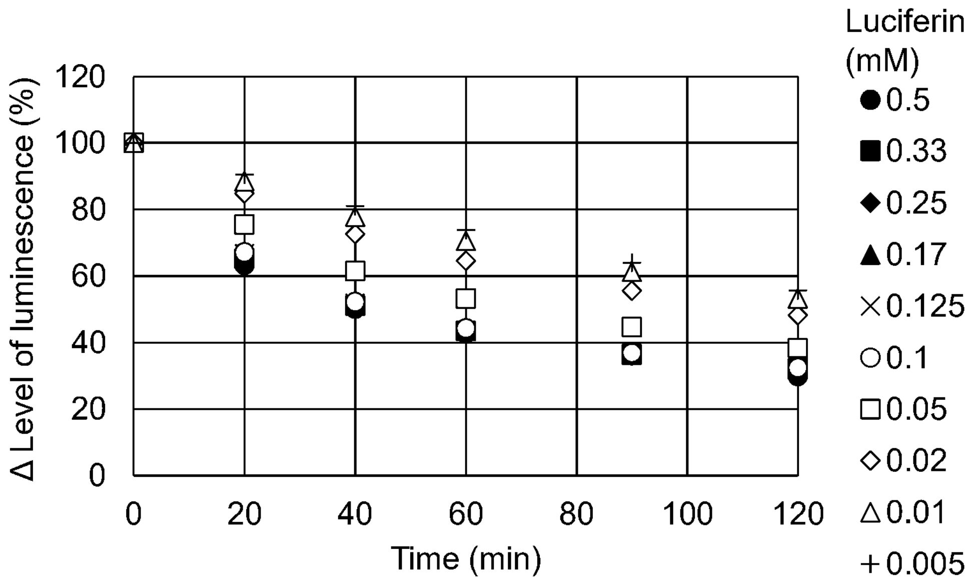
Δ Level of luminescence (%)
120
100
80
60
40
20
0
0
20
40
60
80
100
120
Time (min)
Luciferin (mM)
0.5
0.33
0.25
0.17
0.125
0.1
0.05
0.02
0.01
0.005

Fig. 10

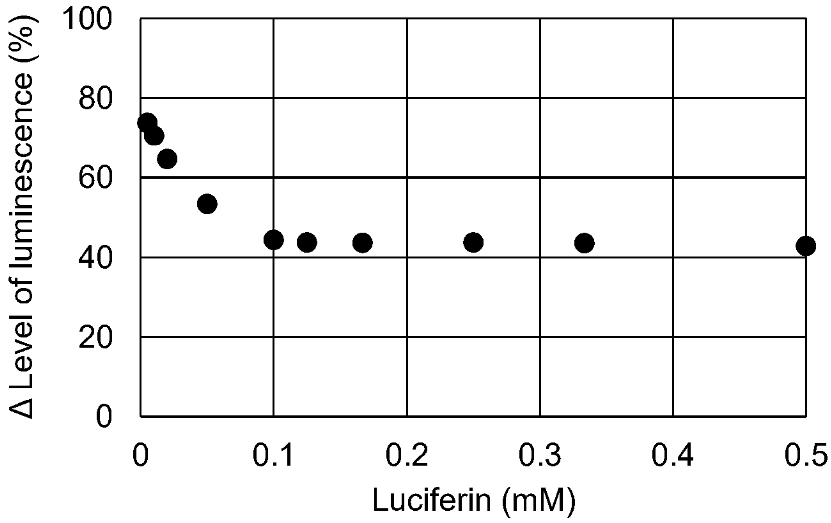
Δ Level of luminescence (%)
100
80
60
40
20
0
0
0.1
0.2
0.3
0.4
0.5
Luciferin (mM)

Fig. 11

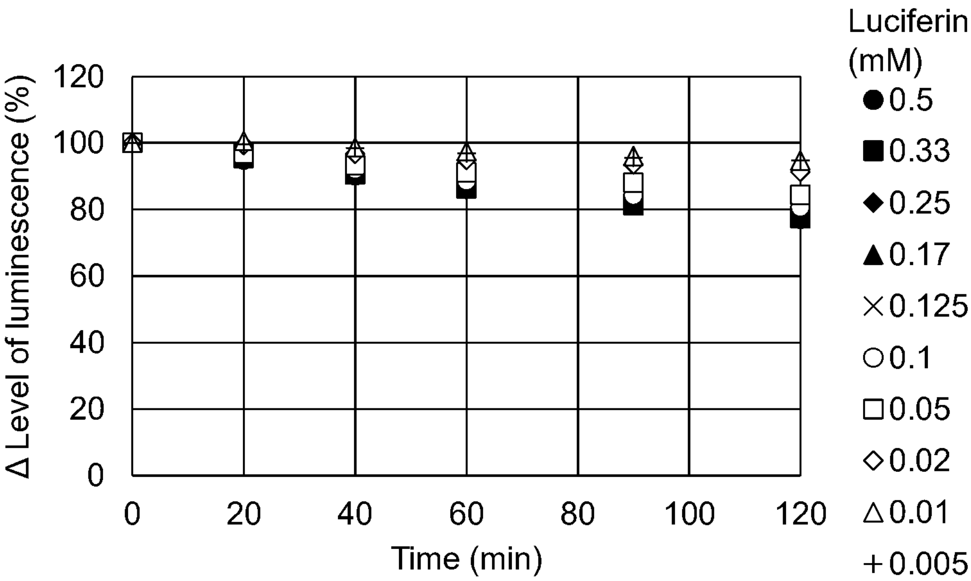
Δ Level of luminescence (%)
120
100
80
60
40
20
0
0
20
40
60
80
100
120
Time (min)
Luciferin (mM)
0.5
0.33
0.25
0.17
0.125
0.1
0.05
0.02
0.01
0.005

Fig. 12

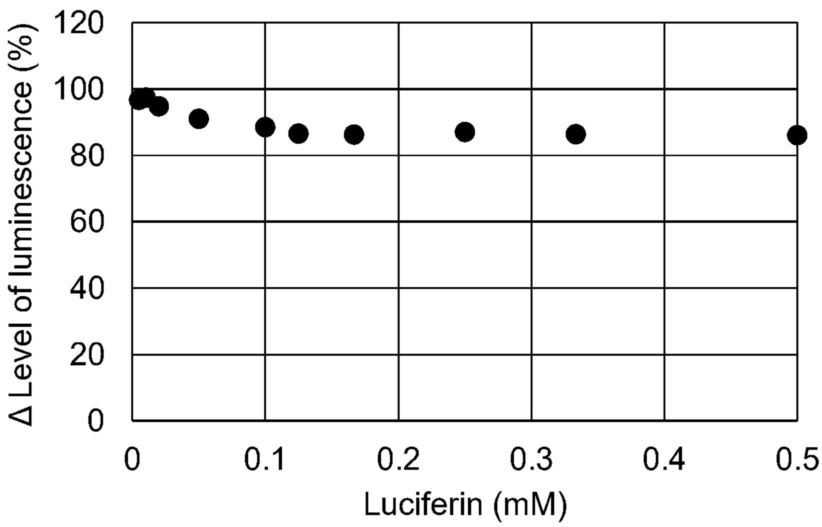
Δ Level of luminescence (%)
120
100
80
60
40
20
0
0
0.1
0.2
0.3
0.4
0.5
Luciferin (mM)

Fig. 13

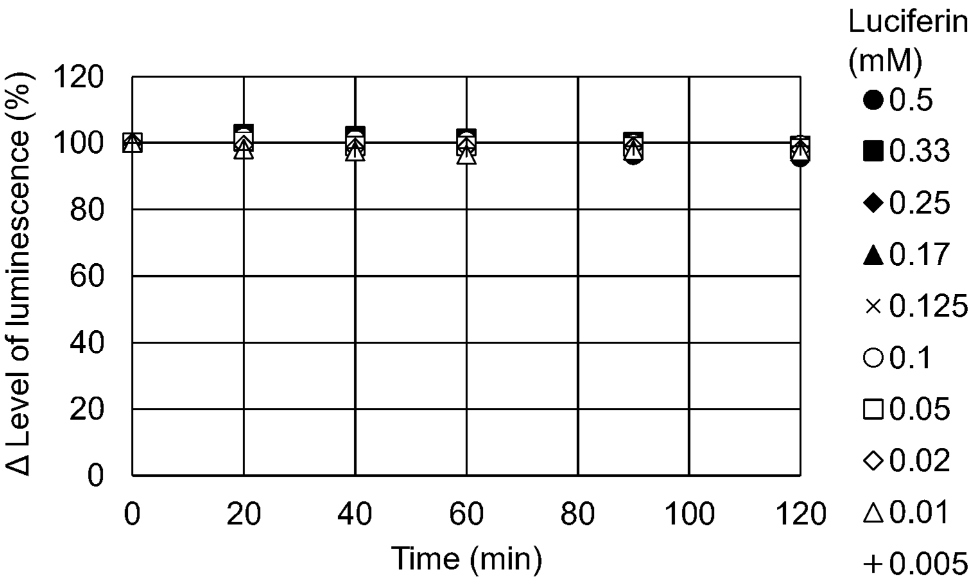
Luciferin
(mM)
0.5
0.33
0.25
0.17
0.125
0.1
0.05
0.02
0.01
0.005
Δ Level of luminescence (%)
120
100
80
60
40
20
0
0
20
40
60
80
100
120
Time (min)

# Fig. 14

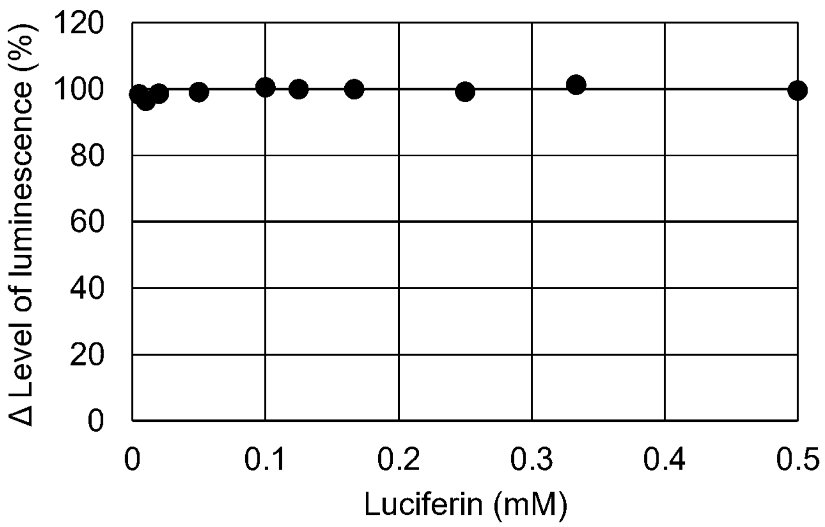
Δ Level of luminescence (%)
120
100
80
60
40
20
0
0
0.1
0.2
0.3
0.4
0.5
Luciferin (mM)

Fig. 15

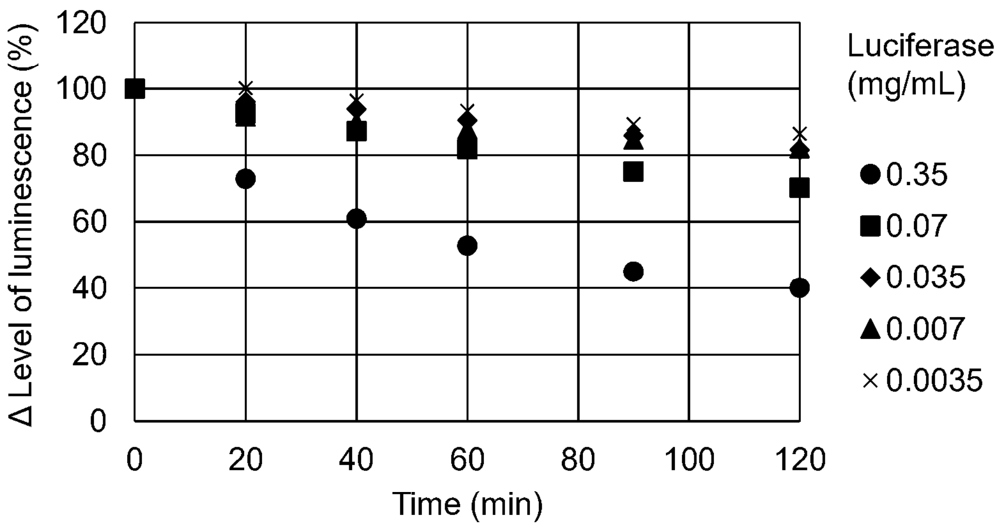
Δ Level of luminescence (%)
120
100
80
60
40
20
0
0
20
40
60
80
100
120
Time (min)
Luciferase (mg/mL)
0.35
0.07
0.035
0.007
0.0035

Fig. 16

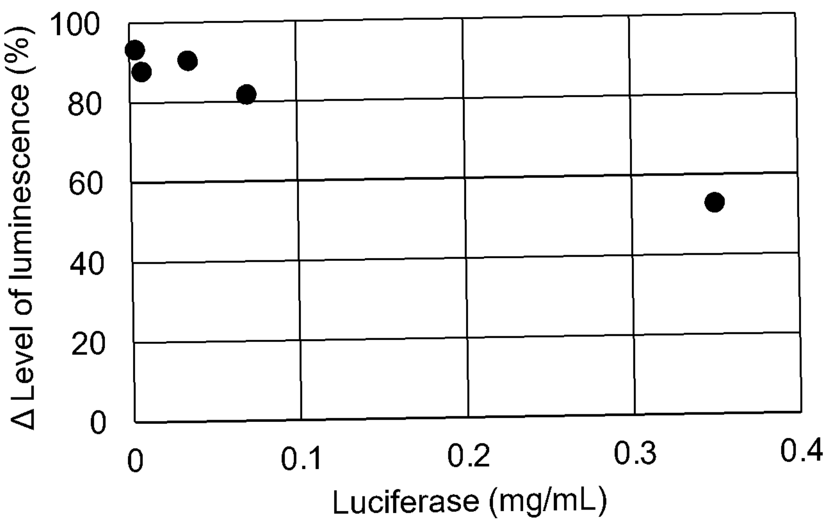
Δ Level of luminescence (%)
100
80
60
40
20
0
0
0.1
0.2
0.3
0.4
Luciferase (mg/mL)

Fig. 17

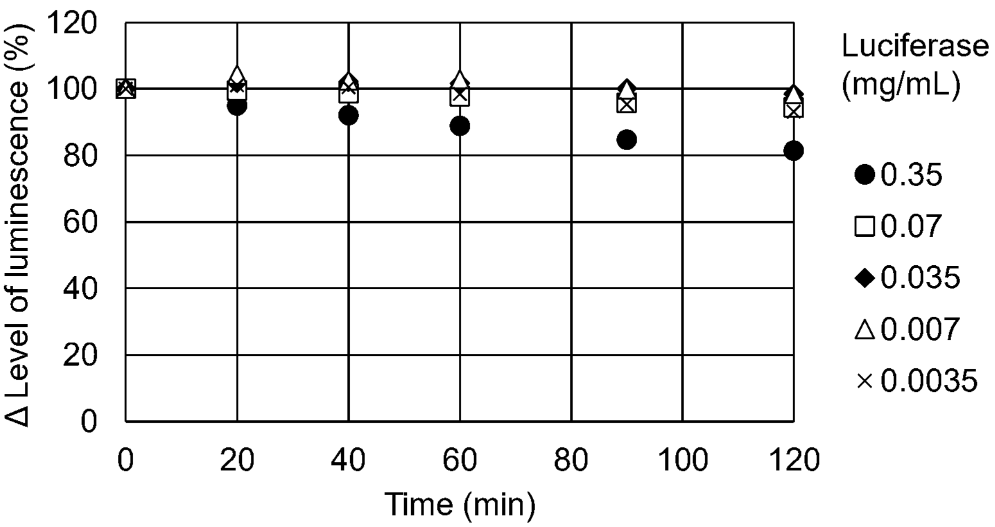
Δ Level of luminescence (%)
120
100
80
60
40
20
0
0
20
40
60
80
100
120
Time (min)
Luciferase (mg/mL)
0.35
0.07
0.035
0.007
0.0035

Fig. 18

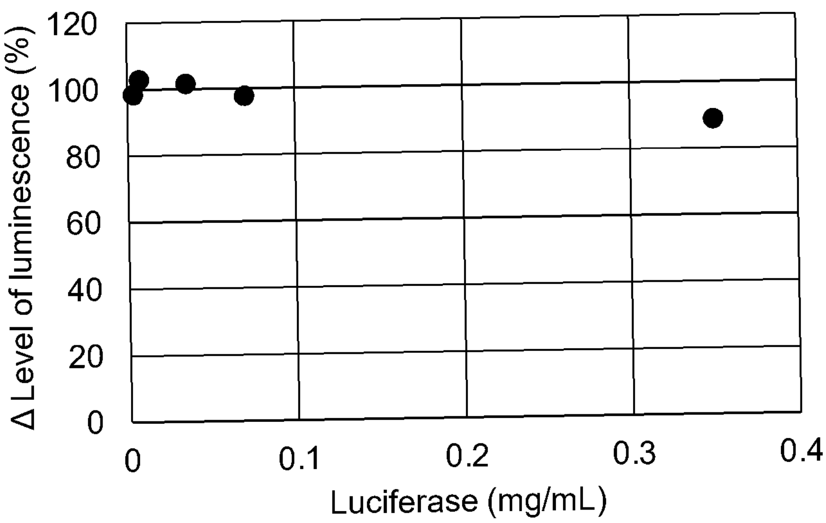
Δ Level of luminescence (%)
120
100
80
60
40
20
0
0
0.1
0.2
0.3
0.4
Luciferase (mg/mL)

Fig. 19

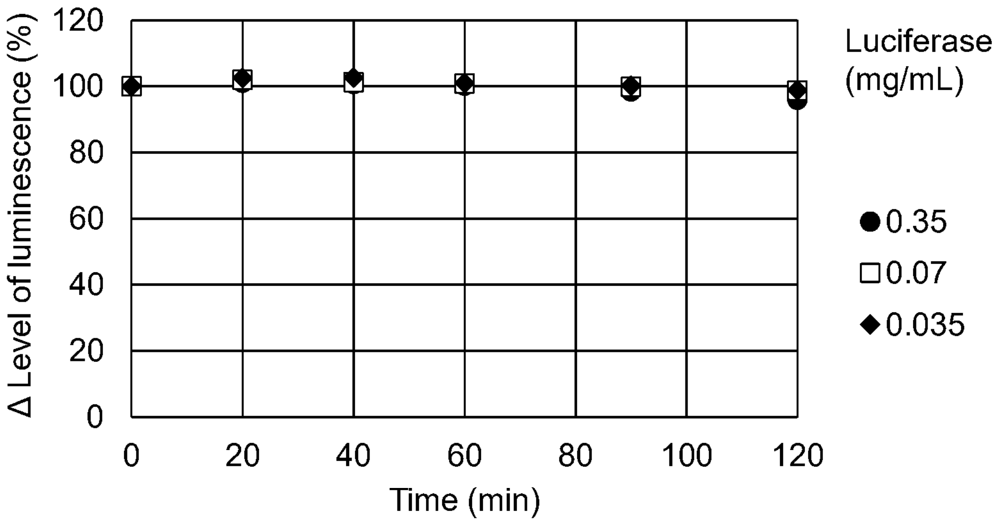
Δ Level of luminescence (%)
120
100
80
60
40
20
0
0
20
40
60
80
100
120
Time (min)
Luciferase (mg/mL)
0.35
0.07
0.035

Fig. 20

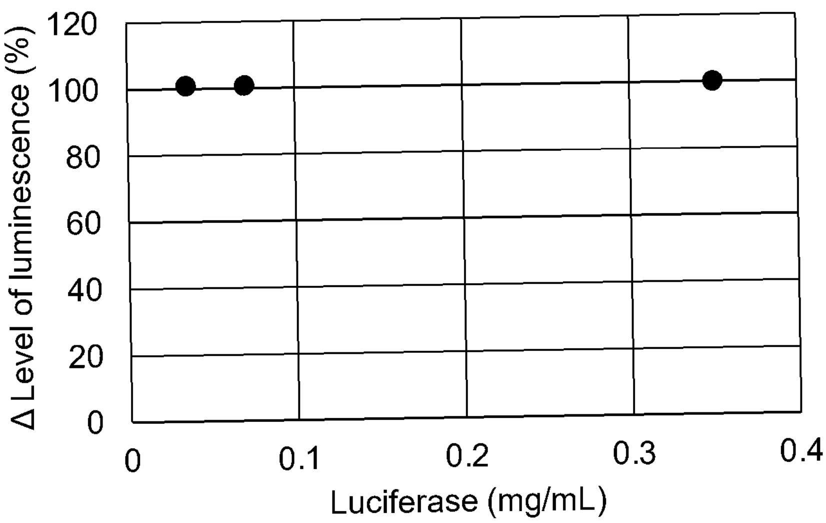
Δ Level of luminescence (%)
120
100
80
60
40
20
0
0
0.1
0.2
0.3
0.4
Luciferase (mg/mL)

Fig. 21

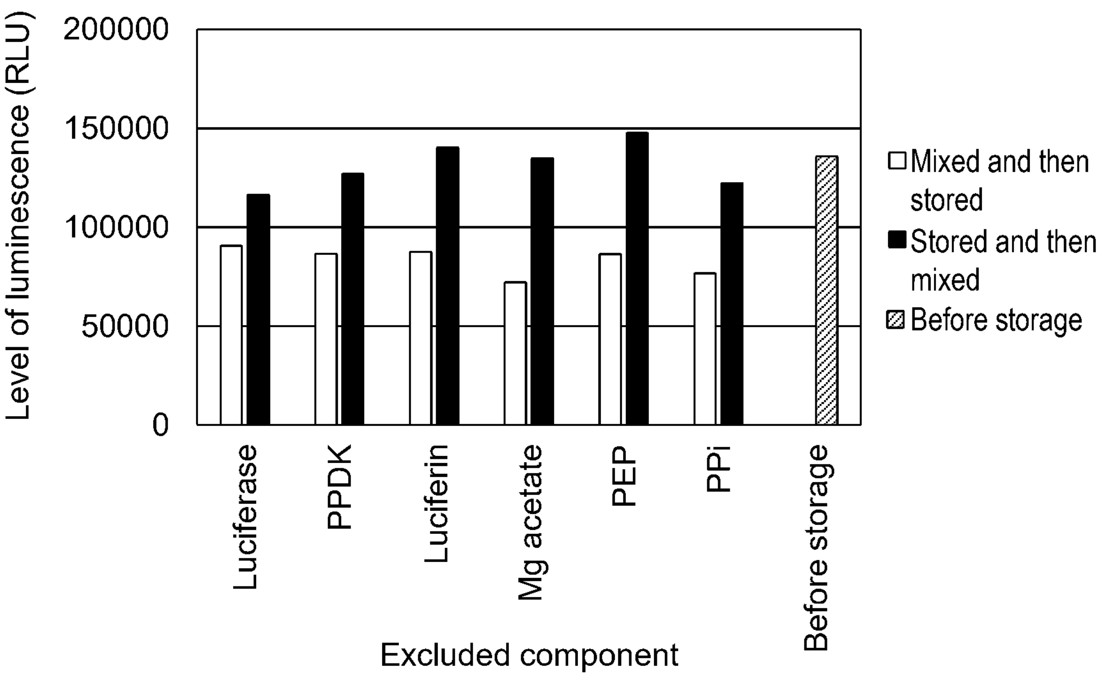
Level of luminescence (RLU)
200000
150000
100000
50000
0
Luciferase
PPDK
Luciferin
Mg acetate
PEP
PPi
Before storage
Excluded component
Mixed and then stored
Stored and then mixed
Before storage

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018147442 A
- WO 2018147443 A
- JP 11239493 A, Kokai
- JP 2011188787 A, Kokai
- JP 2000197484 A, Kokai
- JP 2666561 B
- JP 2003512071 A, Kohyo
- WO 9902697 A
- JP 10512750 A, Kohyo
- JP 2001518799 A, Kohyo
- JP 3048466 B
- JP 2000197487 A, Kokai
- JP 9510610 A, Kohyo
- JP 2003518912 A, Kohyo
- JP 7112434 B, Kokoku
- JP 1051086 A, Kokai
- JP 3749628 B
- JP 2007091695 A, Kokai
- JP 2010523149 A, Kohyo
- JP 2008127677 A
- JP 8168375 A, Kokai


### Non-patent literature cited in the description


- **MARLENE DELUCA** ; **WILLIAM D. MCELROY**. *Biochemistry*, 1974, vol. 13 (5), 921-925